# EUROPEAN PATENT SPECIFICATION

(11) **EP 1 976 529 B1**
(45) Date of publication and mention of the grant of the patent: **11.12.2013**
(21) Application number: 07762831.1
(22) Date of filing: 19.01.2007
(51) Int. Cl.: A61K 31/435, A61K 38/16, A61P 35/00

(54) **ANG2 AND VEGF INHIBITOR COMBINATIONS**
ANG2- UND VEGF-HEMMERKOMBINATIONEN
COMBINAISONS D'INHIBITEURS D'ANG2 ET DE VEGF

(30) Priority: 27.01.2006 US 762493 P
(43) Date of publication of application: 08.10.2008
(73) Proprietor: Amgen Inc., Thousand Oaks, CA 91320-1799 (US)
(72) Inventor: OLINER, Jonathan, Newbury Park, California 91320 (US); KENDALL, Richard, Thousand Oaks, California 91360 (US); KUMAR, Rakesh, Phoenixville, Pennsylvania 19460 (US)
(74) Representative: Care, Alison
(86) International application number: PCT/US2007/001365
(87) International publication number: WO 2007/089445

(56) References cited:
- EP-A- 1 586 333
- WO-A1-2007/068895
- WO-A2-03/083117
- WO-A2-2004/092215
- US-A1- 2003 125 339
- US-A1- 2003 225 106
- US-A1- 2004 147 449
- US-A1- 2005 009 625
- HERBST R ET AL: "151 AMG 706 first in human, open-label, dose-finding study evaluating the safety and pharmacokinetics (PK) in subjects with advanced solid tumors" EUROPEAN JOURNAL OF CANCER. SUPPLEMENT, PERGAMON, OXFORD, GB, vol. 2, no. 8, 1 September 2004 (2004-09-01), page 48, XP004639595 ISSN: 1359-6349
- "1495 PUBLICATION Pharmacokinetics (PK) of AMG 706 on CYP3A activity using oral midazolam and the bioavailability of solid formulations in patients (pts) with advanced solid tumors: results of two phase 1 substudies" EUROPEAN JOURNAL OF CANCER. SUPPLEMENT, PERGAMON, OXFORD, GB, vol. 3, no. 2, 1 October 2005 (2005-10-01), page 433, XP005133605 ISSN: 1359-6349

## Description

This application claims the benefit of U.S. Provisional Application No. 60/762,493 filed January 27, 2006 which is hereby incorporated by reference herein.

### Field of the Invention

The invention relates to combinations of Ang2 inhibitors and VEGF inhibitors and to their use in formulating pharmaceutical compositions and in treating diseases, among other things.

### Background of the Invention

Certain diseases are known to be associated with deregulated angiogenesis, for example ocular neovascularization, such as retinopathies (including diabetic retinopathy), age-related macular degeneration, psoriasis, hemangioblastoma, hemangioma, arteriosclerosis, inflammatory disease, such as a rheumatoid or rheumatic inflammatory disease, especially arthritis (including rheumatoid arthritis), or other chronic inflammatory disorders, such as chronic asthma, arterial or post-transplantational atherosclerosis, endometriosis, and neoplastic diseases, for example so-called solid tumors and liquid tumors (such as leukemias).

For instance, angiogenesis plays a crucial role in tumor progression. Nascent and small tumors can obtain sufficient oxygen and nutrients to sustain their growth by simple diffusion. Beyond a diameter of 1 to 2 mm, however, diffusion cannot provide these elements in the amounts required for further growth. For growth beyond that size, all tumors require a vasculature, whatever their cause, origin, type, age, or location. Thus, tumor growth beyond a diameter of 1 to 2 mm requires angiogenesis. Angiogenesis, accordingly, has been seen as a promising target for developing an effective general treatment for tumors.

Three principal mechanisms play an important part in the activity of angiogenesis inhibitors against tumors: (i) inhibition of the growth of vessels, especially capillaries, into avascular resting tumors, with the result that there is no net tumor growth owing to the balance that is achieved between cell death and proliferation; (ii) prevention of the migration of tumor cells owing to the absence of blood flow to and from tumors; and (iii) inhibition of endothelial cell proliferation, thus avoiding the paracrine growth-stimulating effect exerted on the surrounding tissue by the endothelial cells which normally line the vessels. See R. Connell and J. Beebe, Exp. Opin. Ther. Patents, 11:77-114 (2001).

Efforts to develop therapeutically effective inhibitors of angiogenesis have targeted all three of these principal mechanisms. As a result of these efforts, a variety of promising anti-angiogeneic agents have been identified. While some of these exhibit notable inhibitory effects, none of them has proven to be an entirely satisfactory way to provide a monotherapeutic modality for most angiogenic dependent disorders.

Efforts to obtain a more effective therapeutic profile have focused on identifying new inhibitors, modifying known inhibitors, improving formulation and administration, and using the inhibitors together with other therapeutic modalities. Thus far, while there has been some progress, there is much that needs to be done.

One approach to developing a more effective treatment modality has been to combine agents that act on different targets, preferably targets that act on well isolated pathways. A variety of combinations have been suggested.

Since the Tie2 pathway has been shown to be important to angiogenesis, there have been a few suggestions to try combining a Tie2 pathway inhibitor with a VEGF pathway inhibitor. See Siemeister G., et al., Cancer Research, 59:3185-3191 (1999); Jendreyko, N., et al., Journal of Biological Chemistry, 278:47812-47819 (2003); Jendreyko, N., et al., PNAS, 102:8293-8298 (2005). There is some evidence that the two pathways act independently of one another. However, this does not mean that combining an agent that acts on one pathway with an agent that acts on the other pathway will have an additive effect. Indeed, it is just as likely that at optimum dosage either one of the agents (used alone) will be at least as effective as when used in combination. EP1586333 (Schering Aktiengesellschaft) relates to compositions of compounds that interfere with VEGF/VEGF receptor systems and Angiopoeitin/Tie receptor systems. US 2003/0125339 (Chen et al) relates to heterocyclic compounds for the treatment of angiogenesis mediated diseases. Herbst et al (2004 "151 AMG706 first in human, open label, dose finding study evaluating safety and pharmacokinetics (PK) in subjects with advanced solid tumours" European Journal of Cancer, Supplement, vol 2, no. 8) discuss the safety and dosage levels of AMG706 when tested in patients with solid tumours.

The inventors of the inventions described herein have examined the therapeutic profiles of a variety of combinations of agents that act on the VEGF pathway with agents that act on the Tie2 pathway. They have found that some combinations of a VEGF pathway inhibitor and an Ang2 pathway inhibitor are of particular enhanced utility in treatment of patients.

### Summary of the Invention

The following numbered paragraphs describe a few illustrative embodiments of the invention that exemplify some of its aspects and features. They are not exhaustive in illustrating its many aspects and embodiments, and thus are not in any way limitative of the invention. Many other aspects, features, and embodiments of the invention are described herein. Many other aspects and embodiments will be readily apparent to those skilled in the art upon reading the application and giving it due consideration in the full light of the prior art and knowledge in the field.

The numbered paragraphs below are self-referential. The phrase "according to any of the foregoing or the following" refers to all of the preceding and all of the following numbered paragraphs and their contents. Likewise, the phrase "according to any of the foregoing" refers to all preceding numbered paragraphs and their contents. The phrase "according to any of the following" refers to all of the numbered paragraphs that follow. All phrases of the form "according to #" are direct references to that numbered paragraph, e.g., "according to 46." means according to paragraph 46. in this collection of numbered paragraphs. All cross-references are combinatorial, except for redundancies and inconsistencies of scope. The cross-references are used explicitly to provide a concise description showing the inclusion of the various combinations of subject matter with one another.

The present invention relates to a combination of (a)2xCon4(C), and (b) AMG 706 and pharmaceutically acceptable salts thereof; for use in treating a solid tumour in a subject.

The invention also relates to pharmaceutically acceptable formulations of 2xCon4(C), in combination with AMG 706 for use as above. Preferably, the solid tumour is adenocarcinoma, and more preferably the adenocarcinoma is colon cancer. The subject may be human. 2xCon4(C) and AMG706 may be administered at different times, sequentially. The combination of 2xCon4(C) and AMG706 may be administered at the same time. The AMG 706 may be administered at a dose of 25 mg to 125 mg once a day.

2xCon4(C), is described in WO2004/092215A2 or WO03/05134A2 particularly as to 2xCon4(C);

AMG 706, is described in US2003/0125339 or US. Pat. No. 6995162 or US. Pat. No. 6878714, particularly in parts disclosing AMG 706.

### Brief Descriptions of the Figures

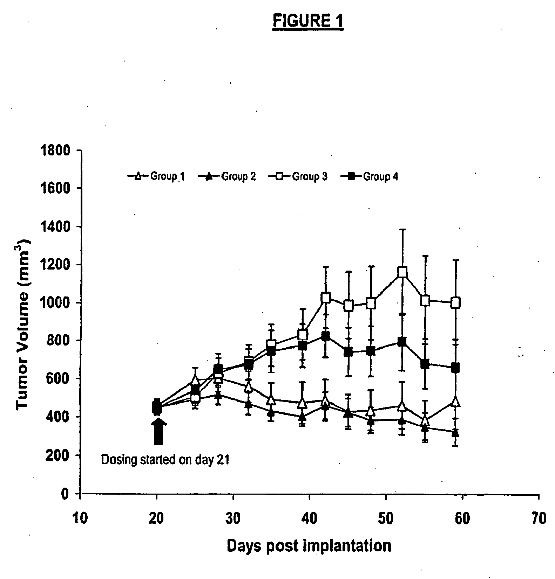

FIGURE 1 graphically presents tumor volumes (mean ± SEM) over the course of the study described in Example 1, in which Colo205 colorectal cancer cell xenograft tumors in female CD1 Nu/Nu mice were treated with Tek472/Fc plus IgG (group 4), Avastin® plus Fc (group 1), Tek472/Fc and Avastin® (group 2), or IgG plus Fc (negative control) (group 3).

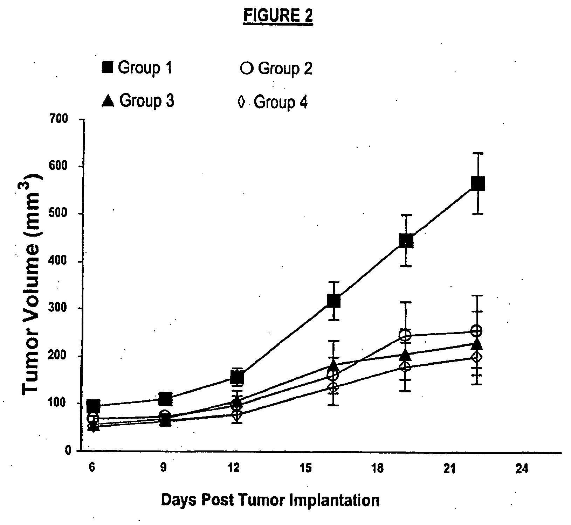

FIGURE 2 graphically presents tumor volumes over the course of the study described in Example 2, in which A431 epidermoid carcinoma cell xenograft tumors in female CD1 Nu/Nu mice were treated with: (1) IgG1 (control) (group 1); (2) anti-Ang2 antibody (Ab 536) and control (group 2); (3) anti-VEGF antibody and control (group 3); and (4) both anti-VEGF antibody and anti-Ang2 antibody (Ab 536) (group 4). Each data point is the mean for a group. Each bar is ± SEM.

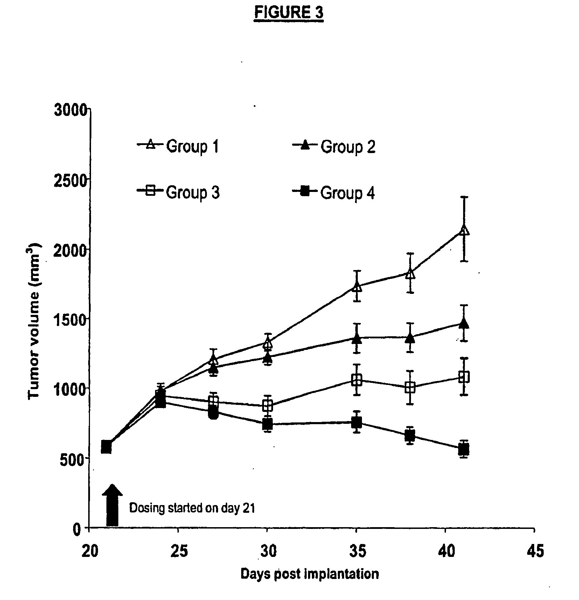

FIGURE 3 graphically depicts tumor volumes over the course of the study described in Example 3, in which HT29 human colon carcinoma cell xenograft tumors in female athymic nude mice were treated with combinations of: (1) vehicle and Fc (group 1); (2) vehicle and 2xCon4(C) (group 2); (3) AMG 706 and Fc (group 3); and (4) AMG 706 and 2xCon4(C) (group 4). Each data point is the mean for a group of ten mice. Each bar represents the range of the SEM.

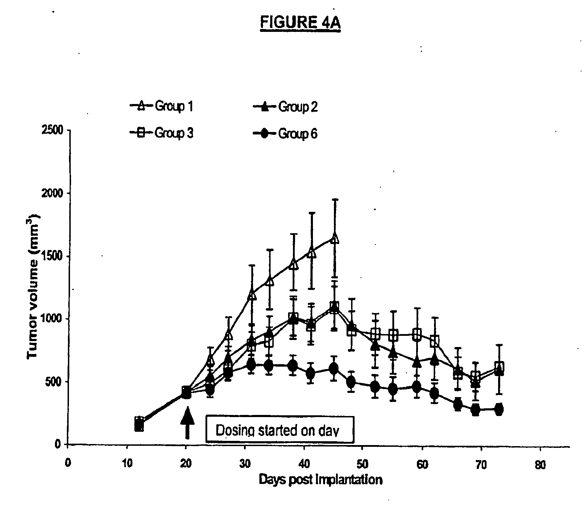

FIGURE 4A through 4D graphically present results of the study described in Example 4, in which Colo205 colorectal cancer cell xenograft tumors in female CD1 Nu/Nu mice were treated with nine combinations of control vehicles, 2xCon4(C), and/or 4TBPPAPC, as set forth in Table 4A and Table 4B. Data represent the mean ± SEM (n=10 mice each).
Figure 4A graphically depicts mean tumor volumes over the course of treatment for groups 1, 2, 3, and 6 which were treated with control vehicles (group 1), a suboptimal dose of 4TBPPAPC (group 2), a suboptimal dose of 2xCon4(C) (group 3), and a suboptimal dose of 4TBPPAPC and a suboptimal dose of 2xCon4(C) (group 6).

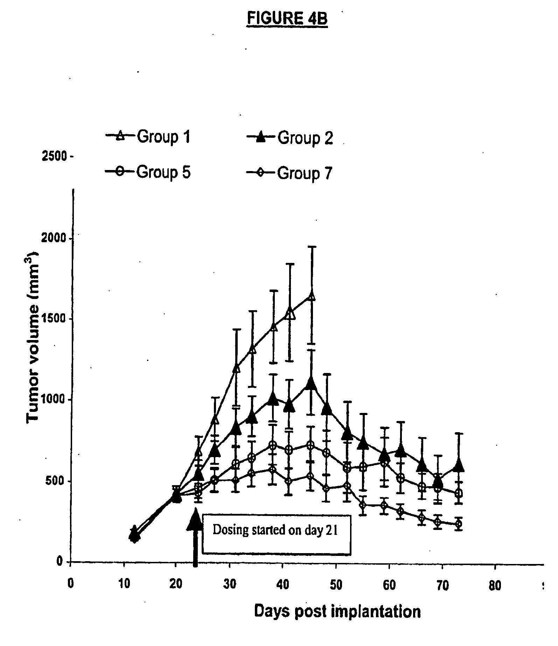

Figure 4B graphically depicts mean tumor volumes over the course of treatment for groups 1, 2, 5, and 7 which were treated with control vehicles (group 1), a suboptimal dose of 4TBPPAPC (group 2), an optimal dose of 2xCon4(C) (group 5), and a suboptimal dose of 4TBPPAPC and an optimal dose of 2xCon4(C) (group 7).

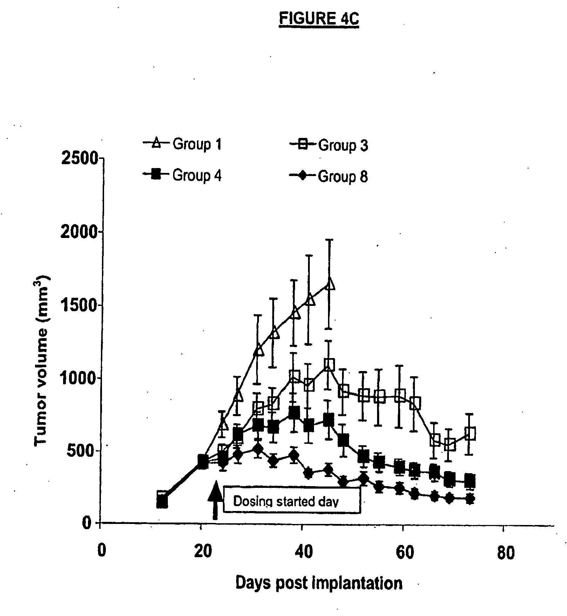

Figure 4C graphically depicts mean tumor volumes over the course of treatment for groups 1, 3, 4, and 8 which were treated with control vehicles (group 1), suboptimal dose of 2xCon4(C) (group 3), an optimal dose of 4TBPPAPC (group 4), and an optimal dose of 4TBPPAPC and a suboptimal dose of 2xCon4(C) (group 8).

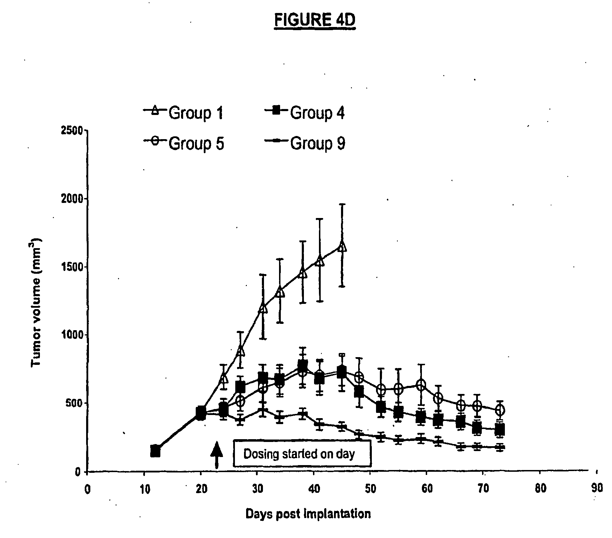

Figure 4D graphically depicts mean tumor volumes over the course of treatment for groups 1, 4, 5, and 9 which were treated with control vehicles (group 1), an optimal dose of 4TBPPAPC (group 4), an optimal dose of 2xCon4(C) (group 5), and an optimal dose of 4TBPPAPC and an optimal dose of 2xCon4(C) (group 9).

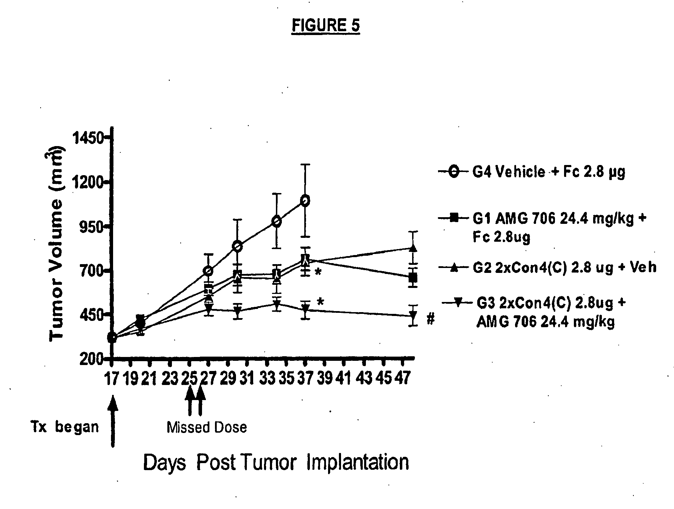

FIGURE 5 graphically depicts mean tumor volumes over the course of treatment of Colo205 colorectal cancer cell xenografts in CD1 Nu/Nu mice treated with AMG 706 (group 1), 2xCon4(C) (group 2), both AMG 706 and 2xCon4(C) (group 3), and FC/vehicle (group 4), as described in Example 5.

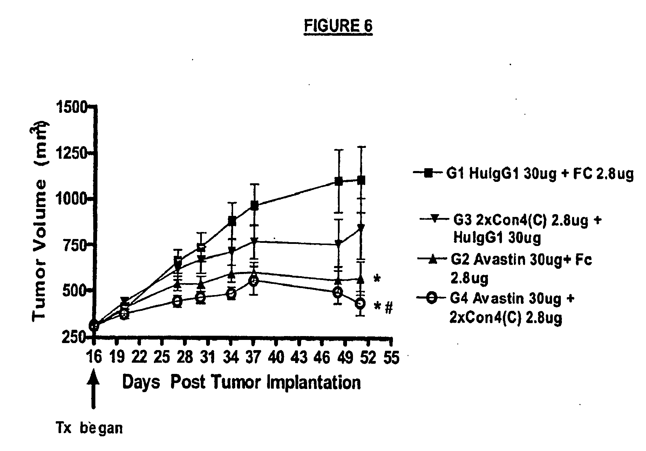

FIGURE 6 graphically depicts mean tumor volumes over the course of treatment of Colo205 colorectal cancer cell xenografts in CD1 Nu/Nu mice treated with 2xCon4(C) (group 3), Avastin™ (group 2), both 2xCon4(C) and Avastin™ (group 4), and control (group 1), as described in Example 6.

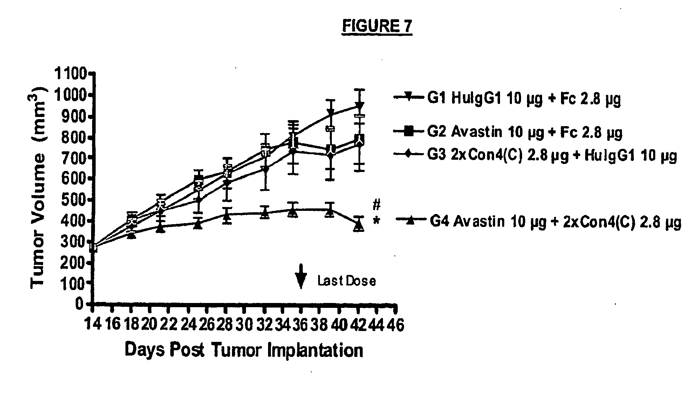

FIGURE 7 graphically depicts mean tumor volumes over the course of treatment of Colo205 colorectal cancer cell xenografts in CD1 Nu/Nu mice treated with 2xCon4(C) (group 3), Avastin™ (group 2), both 2xCon4(C) and Avastin™ (group 4), and control (group 1), as described in Example 7.

### Definitions

Illustrative meanings of certain terms and phrases as used herein are set forth below.

"2xCon4(C)" is an Ang2 selective peptibody, as described in WO03/057134A2 and WO2004/092215A2, particularly in parts pertinent to 2xCon4(C), its structure and properties, methods for making and using it, and other related compounds. 2xCon4(C) is a fusion of a human Fc fragment and two copies of an Ang2 specific binding peptide. 2xCon4(C) is also referred to as 2xCon4(C) 1K. Among other names for 2xCon4(C) are those set forth in the foregoing WIPO publications.

"4TBPPAPC" is a multi-kinase inhibitor that interferes with the Kit, PDGR, and VEGF-signalling pathways. Its name is N-(4-(1,1-dimethylethyl)phenyl)-2-((4-pyridinylmethy)amino-3-pyridinecarboxamide, as described in US Patent Application US2003/0125339, particularly in parts pertinent to 4TBPPAPC, its structure and properties, methods for making and using it, and other related compounds. 4TBPPAPC also is named N-[4-(tert-butyl)phenyl]{2-[(4-pyridylmethy-1)amino](3-pyridyl)}carboxamide.

"A" or "an" means herein one or more than one; at least one. Where the plural form is used herein, it generally includes the singular. The term "compounds" includes one compound, as well as many compounds. The term "salts" includes one salt, as well as many salts.

"Ab 536" is a human Ang2 specific antibody comprising the 536 HC heavy chain and the 536 kappa light chain, as described in WO03/030833 and US Application No. 10/982,440, particularly in parts pertinent to Ab 536, its structure and properties, methods for making and using it, and other related antibodies. Ab 536 and related antibodies and the like are also described in WO04/092215, particularly in parts pertinent to Ab 536 and related peptides and proteins, their structures and properties, and methods for making and using them.

"Ang" is a designation for angiopoietin.

"Ang2" is a designation for angiopoietin 2.

"Ang2 inhibitor" as used herein is any substance that decreases the effective activity of Ang2 in a given circumstance. Ang2 inhibitors can be, to name just a few examples, small molecules, peptides, polypeptides, proteins, including more specifically antibodies, including anti-Ang2 antibodies, intrabodies, maxibodies, minibodies, diabodies, Fc fusion proteins such as peptibodies, receptibodies, soluble Tie-2 receptor proteins and fragments, and a variety of others. Many Ang2 inhibitors work by binding to Ang2. Others work by binding to factors that bind to or are bound by Ang2. Other Ang2 inhibitors act more indirectly, for instance by altering regulatory posttranslational modifications such as phosphorylation that control the signaling engendered by Ang2 or by altering the interaction of Ang2 with other factors. Ang2 inhibitors in accordance with the invention also may act in more indirect ways to decrease Ang2 activity. Whatever the mechanism, as used herein, an Ang2 inhibitor decreases the effective activity of Ang2 in a given circumstance over what it would be in the same circumstance in the absence of the inhibitor.

"Avastin™" is a recombinant humanized monoclonal antibody that binds directly to VEGF, and is sold by Genentech. It is also called bevacizumab, R-435, rhuMAB-VEGF, and CAS Registry Number 216974-75-3.

"BAY 43-9006" - see Nexavar®.

"Cancer" and "cancerous" refer to or describe the physiological condition in mammals that is typically characterized by unregulated cell growth. Examples of cancer include, but are not limited to, carcinoma, lymphoma, sarcoma, blastoma, and leukemia. More particular examples of such cancers include squamous cell carcinoma, lung cancer, pancreatic cancer, cervical cancer, bladder cancer, hepatoma, breast cancer, colon carcinoma, and head and neck cancer.

"Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of" and similar phrases using words of the root "comprise" are used herein as synonyms of "comprising" and have the same meaning.

"Comprised of" is a synonym of comprising (see above).

"Deleterious" means, as used herein, harmful. By way of illustration, "deleterious" processes include, for example, harmful effects of disease processes and harmful side effects of treatments.

"Dysfunction" means, as used herein, a disorder, disease, or deleterious effect of an otherwise normal process.

"Effective amount" generally means an amount which provides the desired local or systemic effect. For example, an effective amount is an amount sufficient to effectuate a beneficial or desired clinical result. The effective amounts can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"FBS" means fetal bovine serum.

"Kit" means a collection of items used together for a given purpose or purposes, in particular in the context of the present disclosure, a set of items in one (or more than one package) for use with one another for combination therapy comprising, for instance, 2xCon4(C) and one or both of AMG 706 and 4TBPPAPC.

"Modality" means a type, approach, avenue, or method, such as a therapeutic modality; i.e., a type of therapy.

"AMG 706" is a multi-kinase inhibitor that interferes with the Kit, PDGF, and VEGF-signalling pathways, as described in US2003/0125339, particularly in parts pertinent to AMG 706, its structure and properties, methods for making and using it, and other related compounds. Its chemical name is N-(2,3-dihydro-3,3-dimethyl-1H-indol-6-yl)-2-[(4-pyridinylmethyl)amino]-3-pyridinecarboxamide (I). As used herein the term AMG 706 includes pharmaceutically acceptable salts, in particular, the diphosphate salt, except as otherwise provided herein. AMG 706 is also known as motesanib diphosphate.

"Nexavar®" (also known as BAY 43-9006, sorafenib tosylate, CAS Registry Number 284461-73-0, raf kinase inhibitor, sorafenib analogs, and IDDBCP150446, among others) is a substituted omega carboxy diphenyl urea that inhibits RAF-1 activation, and thereby decreases RAF-1 dependent phosphorylation of MEK-1 and ERK-1, as described in US Patent Application No. 2003/0125359A1, WO03/047523A2, and Wilhelm et al., Current Pharmaceutical Design, vol. 8, pp. 2255-2257 (2002), particularly in parts pertinent to Nexavar®, its structure and properties, methods for making and using it, and other related molecules. Its chemical name is 4-(4-{3-[4-Chloro-3-(trifluoromethyl)phenyl]ureido}phenoxy)-N²-methylpyridine-2-carboxamide. A variety of derivatives have been produced. Among these are fluorinated derivatives described in US Patent Application 2005/0038080A1 and WO2005/009961 A2, particularly as to these and other pharmaceutically active diphenyl urea compounds.

"Optimum dose" means a specific dose or a range of doses that provide the best outcome under the particular circumstances and for the purpose of its administration.

"PBS" means phosphate buffered saline.

"Peptibody" refers to a molecule comprising an antibody Fc domain attached to at least one peptide. The production of peptibodies is generally described in PCT publication WO 00/24782, published May 4, 2000, particularly as to the structure, synthesis, properties, and uses of peptibodies, and to the same specifically regarding Ang2 inhibitors and VEGF inhibitors.

"Pharmaceutically acceptable derivative" is any pharmaceutically acceptable derivative of a compound of the invention, such as a salt, an ester, a metabolite, or a residue of a compound of this invention.

"PTK/ZK," also known as vatalanib, is a multi-VEGF receptor tyrosine kinase inhibitor that is said to block tumor angiogenesis and lymphangiogenesis. Its chemical name is N-(4-chlorophenyl)-4-(pyridin-4-ylmethyl)phthalazin-1-amine. It also is known as CAS Registry Numbers 212141-54-3 and 212142-18-2, PTK787, PTK787/ZK, PTK-787/ZK-222584, PTK787/ZK222584, ZK-22584, VEGF-TKI, VEGF-RKI, PTK-787A, DE-00268, CGP-79787, CGP-79787D, vatalanib, and ZK-222584. See Thomas, A., et al., J. of Clin. Oncology, 23(18): 4162-4171 (2005); US Patent Application 20050118600A1, particularly as to the structure, synthesis, properties, and uses of PTK/ZK and related compounds.

"SEM" means standard error of the mean.

"Subject" means a vertebrate, such as a mammal, such as a human. Mammals include, but are not limited to, humans, farm animals, sport animals, and pets.

Subjects in need of treatment by methods and/or compositions of the present invention include those suffering from a disorder, dysfunction, or disease, or a side effect thereof, or from a side effect of a treatment thereof.

"Sutent®" is a small molecule receptor tyrosine kinase inhibitor with the chemical name (5-[5-fluoro-2-oxo-1,2- dihydroindol-(3Z)-ylidenemethyl]-2, 4-dimethyl-1*H*-pyrrole-3-carboxylic acid [2-diethylaminoethyl]amide). Sutent® is also known as sunitinib malate, SU11248, SU-11248, SU-011248, and SU-11248J, and is reported to have anti-angiogenic and anti-tumor activities. See Mendel, D., et al., Clinical Cancer Research, 9:327-337 (2003); Schlessinger, J., The Scientist, 19(7):17 (2005), particularly as to the structure, synthesis, properties, and uses of Sutent® and related compounds.

"Tek472/Fc" is a fusion of the N-terminal 472 amino acids of Tek to a 232 amino acid portion of the Fc region of human IgG1, as described in WO00/75323A1, particularly in parts pertinent to Tek472/Fc, its structure and properties, methods for making and using it, and other related fusion polypeptides. The N-terminal Tek fragment constitutes a soluble Tek peptide that is missing fibronectin type III motifs. It has a much higher affinity for Tek ligands than other Tek polypeptides that comprise full-length extra-cellular domains.

"Therapeutically effective" generally is used to qualify the amount of an agent to encompass those amounts that achieve an improvement in disorder severity. For example, effective neoplastic therapeutic agents prolong the survivability of the patient, inhibit the rapidly-proliferating cell growth associated with the neoplasm, or effect a regression of the neoplasm. Treatments that are therapeutically effective within the meaning of the term as used herein, include treatments that improve a subject's quality of life even if they do not improve the disease outcome per se.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

"VEGF inhibitor" as used herein is any substance that decreases signaling by the VEGF-VEGFR pathway. VEGF inhibitors can be, to name just a few examples, small molecules, peptides, polypeptides, proteins, including more specifically antibodies, including anti-VEGF antibodies, anti-VEGFR antibodies, intrabodies, maxibodies, minibodies, diabodies, Fc fusion proteins such as peptibodies, receptibodies, soluble VEGF receptor proteins and fragments, and a variety of others. Many VEGF inhibitors work by binding to VEGF or to a VEGF receptor. Others work more indirectly by binding to factors that bind to VEGF or to a VEGF receptor or to other components of the VEGF signaling pathway. Still other VEGF inhibitors act by altering regulatory posttranslational modifications that modulate VEGF pathway signaling. VEGF inhibitors in accordance with the invention also may act through more indirect mechanisms. Whatever the mechanism involved, as used herein, a VEGF inhibitor decreases the effective activity of the VEGF signaling pathway in a given circumstance over what it would be in the same circumstance in the absence of the inhibitor.

### Description of the Invention

The inventors of the inventions described herein have examined the therapeutic profiles of various combinations of VEGF inhibitors and Ang2 inhibitors. They have found, in accordance with certain preferred aspects and embodiments of the invention, that combining inhibitors can provide advantages over the use of one or the other alone. By way of introduction to these and other aspects of the invention, a brief review of the VEGF pathway and Ang2 pathway is provided below.

### VEGF Pathway

A central pathway in the network regulating the growth and differentiation of the vascular system and its components, both during embryonic development and normal growth, and in a wide number of pathological anomalies and diseases, is mediated by Vascular Endothelial Growth Factor ("VEGF") (originally termed Vascular Permeability Factor or VPF) and the cellular receptors of VEGF ("VEGFRs"). (See G. Breier et al., Trends in Cell Biology, 6:454-456 (1996)). It has therefore been a focus of study and a target for the development of drugs.

VEGF is a dimeric, disulfide-linked 46-kDa glycoprotein related to Platelet-Derived Growth Factor ("PDGF"). It is produced by normal cell lines and tumor cell lines; is an endothelial cell-selective mitogen; shows angiogenic activity in *in vivo* test systems (e.g., rabbit cornea); is chemotactic for endothelial cells and monocytes; and induces plasminogen activators in endothelial cells, which are involved in the proteolytic degradation of the extracellular matrix during the formation of capillaries. A number of isoforms of VEGF are known, which while they show comparable biological activity, differ in the type of cells that secrete them and in their heparin-binding capacity. In addition, there are other members of the VEGF family, such as Placenta Growth Factor ("PGF") and VEGF-C.

The cellular receptors of VEGFs (VEGFRs) are transmembranous receptor tyrosine kinases. They are characterized by an extracellular domain with seven immunoglobulin-like domains and an intracellular tyrosine kinase domain. Various types of VEGF receptor have been characterized, including VEGFR-1 (also known as flt-1), VEGFR-2 (also known as KDR), and VEGFR-3.

A large number of human tumors, especially gliomas and carcinomas, express high levels of VEGF and VEGFRs. This has led to the hypothesis that VEGF released by tumor cells stimulates the growth of blood capillaries and the proliferation of tumor endothelium in a paracrine manner and, through the improved blood supply, accelerates tumor growth. Increased VEGF expression could explain the occurrence of cerebral edema in patients with glioma.

Direct evidence of the role of VEGF as a tumor angiogenesis factor *in vivo* has been found in studies in which VEGF expression or activity was inhibited. This has been achieved with anti-VEGF antibodies, with dominant-negative VEGFR-2 mutants that inhibited signal transduction, and with antisense-VEGF RNA. All of the approaches led to a reduction in the growth of glioma cell lines or other tumor cell lines *in vivo* as a result of inhibited tumor angiogenesis.

VEGFs contribute to vascular hyperpermeability and the formation of edema. Indeed, vascular hyperpermeability and edema that is associated with the expression or administration of many other growth factors appears to be mediated via VEGF production.
Inflammatory cytokines stimulate VEGF production. Hypoxia results in a marked upregulation of VEGF in numerous tissues. Thus, situations involving infarct, occlusion, ischemia, anemia, or circulatory impairment typically invoke VEGFNPF-mediated responses. Vascular hyperpermeability, associated edema, altered transendothelial exchange and macromolecular extravasation, which is often accompanied by diapedesis, can result in excessive matrix deposition, aberrant stromal proliferation, fibrosis, etc. Hence, VEGF-mediated hyperpermeability can significantly contribute to disorders with these etiologic features. Accordingly, these regulators of angiogenesis have become an important therapeutic target. See Hicklin and Ellis, J. Clin Oncology, 23:1011-1027 (2005).

### Tie2/TEK Pathway

The Tie2 receptor tyrosine kinase (referred to as "Tie2," "Tie2R," "ORK," "Tie2/Tek," and "murine Tek") and its ligands (the angiopoietins) also are known to be critical signaling components in a regulatory pathway of angiogenesis (Gale, N.W. and Yancopoulos, G.D., Genes Dev. 13:1055-1066 (1999)). There are 4 known angiopoietins, angiopoietin-1 ("Ang1") through angiopoietin-4 ("Ang4"), and they are also referred to as "Tie2 ligands." (Davis, S., et al., Cell, 87:1161-1169 (1996); Grosios, K., et al., Cytogenet Cell Genet, 84:118-120 (1999); Holash, J., et al., Investigative Ophthalmology & Visual Science, 42:1617-1625 (1999); Koblizek, T. I., et al., Current Biology, 8:529-532 (1998); Lin, P., et al., Proc Natl Acad Sci USA, 95:8829-8834 (1998); Maisonpierre, P. C., et al., Science, 277:55 60 (1997); Papapetropoulos, A., et al., Lab Invest, 79:213-223 (1999); Sato, T. N., et al., Nature, 375:70- 74 (1998); Shyu, K. G., et al., Circulation, 98:2081-2087 (1998); Suri, C., et al., Cell, 87:1171-1180(1996); Suri, C., et al., Science, 282:468-471 (1998); Valenzuela, D. M., et al., Proc Natl to Acad Sci USA, 96:1904-1909 (1999); Witzenbichler, B., et al., J Biol Chem, 273:18514-18521 (1998)). Ang1 binding to Tie2 has been shown to stimulate receptor phosphorylation in cultured endothelial cells. Ang2 has been shown to be both an agonist and antagonist of Tie2 receptor phosphorylation (Davis, S., et al., (1996), supra; Maisonpierre, P.C., et al., (1997), supra; Kim, I., J.H. Kim, et al., Oncogene 19(39):4549-4552 (2000)).

The phenotypes of mouse Tie2 and Ang1 knockouts are similar and suggest that Ang1-stimulated Tie2 phosphorylation mediates remodeling and stabilization of developing vessels in utero through maintenance of endothelial cell-support cell adhesion (Dumont, D.J., et al., Genes & Development, 8:1897 1909 (1994); Sato, T.N., et al., Nature, 376:70-74 (1995); Suri, C., et al., (1996), supra). The role of Ang1 in vessel stabilization is thought to be conserved in the adult, where it is expressed widely and constitutively (Hanahan, D., Science, 277:48-50 (1997); Zagzag, D., et al., Experimental Neurology, 159:391-400 (1999)). In contrast, Ang2 expression is primarily limited to sites of vascular remodeling, where it is thought to block Ang1 function, thereby inducing a state of vascular plasticity conducive to angiogenesis (Hanahan, D., (1997), supra; Holash, J., et al., Science, 284:1994-1998 (1999); Maisonpierre, P. C., et al., (1997), supra).

A variety of publications have reported on selective expression of Ang2 in vessels in disease states that have an associated angiogenic component. Ang2 has been reported in this regard in psoriasis, macular degeneration, and cancer, for instance (Bunone, G., et al., American Journal of Pathology, 155:1967-1976 (1999); Etch, T., et al., Cancer Research, 61:2145-2153 (2001); Hangai, M., et al., Investigative Ophthalmology & Visual Science, 42:1617-1625 (2001); Holash, J., et al., (1999) supra; Kuroda, K., et al., Journal of Investigative Dermatology, 116:713-720 (2001); Otani, A., et al., Investigative Ophthalmology & Visual Science, 40:1912-1920 (1999); Stratmann, A., et al., American Journal of Pathology, 153:1459-1466 (1998); Tanaka, S., et al., J Clin Invest, 103:34-345 (1999); Yoshida, Y., et al., International Journal of Oncology, 15:1221-1225 15 (1999); Yuan, K., et al., Journal of Periodontal Research, 35: 165- 171 (2000); Zagzag, D., et al., (1999) supra). Most of these studies were directed to neoplasms and, in sum, they establish vascular Ang2 expression in a wide variety of tumor types.

In contrast with its expression in pathological angiogenesis, Ang2 expression in normal tissues is extremely limited (Maisonpierre, P. C., et al., (1997), supra; Mezquita, J., et al., Biochemical and Biophysical Research Communications, 260:492-498 (1999)). Indeed, the three main sites of angiogenesis in a normal adult are the ovary, placenta, and uterus, and these are the three sites in which Ang2 mRNA has been preponderantly detected in non-neoplastic adult tissues.

Functional studies provide further evidence that Ang2 is a factor in tumor-associated angiogenesis. Ahmad et al. (Cancer Res., 61: 1255-1259 (2001)) reported that over-expression of Ang2 in a mouse xenograft model increased tumor growth. Etoh et al., supra, and Tanaka et al., supra, similarly reported that over expression of Ang2 results in tumor hypervascularity. In contrast, Yu et al. (Am. J. Path., 158:563-570 (2001)) reported that overexpression of Ang2 in Lewis lung carcinoma and TA3 mammary carcinoma cells prolonged survival of mice injected with the corresponding transfectants.

As a result it has been suggested that Ang1, Ang2, and/or Tie2 is a possible target for anti-cancer therapy. For example, U.S. Patent Nos. 6,166,185, 5,650,490, and 5,814,464 each disclose the concept of anti-Tie2 ligand antibodies and receptor bodies. Lin et al. (Proc. Natl. Acad. Sci USA, 95:8829-8834 (1998)) injected an adenovirus expressing soluble Tie2 into mice, and reported that it resulted in the production of soluble Tie2 and decreased the number and size of the tumors in the mice. In a related study, Lin et al (J. Clin. Invest., 100:2072-2078 (1997)) injected a soluble form of Tie2 into rats, and concluded that this agent reduced tumor size in the rats. Siemeister et al. (Cancer Res., 59:3185- 3189 (1999)) injected human melanoma cell lines expressing the extra-cellular domain of Tie2 into nude mice and reported the production of soluble Tie2 in the animals that resulted in a "significant inhibition" of tumor growth and tumor angiogenesis.

In fact, while there has been notable progress in developing monotherapeutic agents that target one pathway, as yet no agents have been developed for targeting both pathways that provide better results than the corresponding monotherapies. Furthermore, as illustrated by the examples set forth herein, contrary to the prevailing view based on pathway independence, many combinations of agents that together target both pathways work no better than monotherapies with each of the individual agents.

The invention herein described nonetheless provides combination therapies that can be used to treat diseases with efficacies that match and in some cases can exceed the results obtained using monotherapies. Ang2 inhibitors and VEGF inhibitors useful in combination in accordance with the invention herein described are discussed below.

### Ang2 Inhibitors, VEGF Inhibitors and Combinations Thereof

The invention herein disclosed in various of its preferred aspects and embodiments thereof provides a combination of
(a) 2xCon4(C), and
(b) AMG 706 and pharmaceutically acceptable salts thereof; for use in treating a solid tumour in a subject.

### Ang-2 Inhibitors

Among the Ang2 inhibitors are the following: (a) a non-naturally occurring polypeptide that specifically binds to Ang2, including, but not limited to, peptibodies that specifically bind Ang2; (b) a non-naturally occurring antibody that binds Ang2, comprising one or more heterologous CDRs; and (c) a non-naturally occurring polypeptide comprising a soluble receptor fragment that specifically binds Ang2 and an Fc region of an antibody or a portion thereof.

Among Ang2 inhibitors are the following: (a) a non-naturally occurring peptide or peptibody that specifically binds to Ang2, as described in WO2004/092215A or WO03/05134A2, particularly as to these Ang2 inhibitors; (b) a non-naturally occurring antibody that binds Ang2, and comprises one or more heterologous CDRs, as described in WO03/030833A2 and US Application No. 10/982,440, particularly as to these Ang2 inhibitors; and (c) a non-naturally occurring polypeptide comprising a soluble Tie2/Tek receptor fragment lacking at least part of the region containing the FNIII motifs, as described in WO00/75323A1 and related polypeptides as described in US Patent No. 6,166,185, particularly as to these Ang2 inhibitors.

Among Ang2 inhibitors are the following: (a) 2xCon4(C), as described in WO2004/092215A or WO03/05134A2, particularly as to 2xCon4(C); (b) Ab 536, as described in WO03/030833A2 and US Application No. 10/982,440, particularly as to Ab 536; and (c) Tek472/Fc, as described in WO00/75323A1 or related polypeptides as described in US Patent No. 6,166,185, particularly as to Tek472/Fc.

In this regard, certain Ang2 inhibitors are further described below, among these 2xCon4(C) of the invention.

### (1) 2xCon4(C)

2xCon4(C)is an Ang2 selective peptibody, as described in WO03/057134A2 and WO2004/092215A2, particularly in parts pertinent to 2xCon4(C), its structure and properties, methods for making and using it, and other related compounds. 2xCon4(C) is a fusion of a human Fc fragment and two copies of an Ang2 specific binding peptide. 2xCon4(C) is also referred to as 2xCon4(C) 1K.

### (2) Ab 536

Ab 536 is a human Ang2 specific antibody comprising the 536 HC heavy chain and the 536 kappa light chain, as described in WO03/030833 and US Application No. 10/982,440, particularly in parts pertinent to Ab 536, its structure and properties, methods for making and using it, and other related antibodies.

### (3) Tek472/Fc

Tek472/Fc is a fusion of the N-terminal 472 amino acids of Tek to a 232 amino acid portion of the Fc region of human IgG1, as described in WO00/75323A1, particularly in parts pertinent to Tek472/Fc, its structure and properties, methods for making and using it, and other related fusion polypeptides. The N-terminal Tek fragment constitutes a soluble Tek peptide that is missing fibronectin type III motifs. It has a much higher affinity for Tek ligands than other Tek polypeptides that comprise full-length extra-cellular domains.

### VEGF Inhibitors

A great many VEGF inhibitors have been described in the literature. In addition to those described in further detail below, VEGF inhibitors are described in the following patent documents: US 2003/0105091, US2006/0241115, US 5,521,184, US 5,770,599, US 5,990,141, US 6,235,764, US 6,258,812, US 6,515,004, US 6,630,500, US 6,713,485, WO2005/070891, WO 01/32651, WO 02/68406, WO 02/66470, WO 02/55501, WO 04/05279, WO 04/07481, WO 04/07458, WO 04/09784, WO 02/59110, WO 99/450029, WO 00/59509, WO 99/61422, WO 00/12089, WO 00/02871, and WO 01/37820, particularly in parts pertinent to VEGF inhibitors.

The following are among specific VEGF inhibitors:
ABT-869 (Abbott) including formulations for oral administration and closely related VEGF inhibitors;
AEE-788 (Novartis) (also called AE-788 and NVP-AEE-788, among others) including formulations for oral administration and closely related VEGF inhibitors;
AG-13736 (Pfizer) (also called AG-013736) including formulations for oral administration and closely related VEGF inhibitors;
AG-028262 (Pfizer) and closely related VEGF inhibitors;
Angiostatin (EntreMed) (also called CAS Registry Number 86090-08-6, K1-4, and rhuAngiostatin, among others) and closely related inhibitors as described in, among others, US Patent Nos. 5,792,825 and 6,025,688, particularly in parts pertaining to Angiostatin and closely related VEGF inhibitors, their structures and properties, and methods for making and using them;
Avastin™ (Genentech) (also called bevacizumab, R-435, rhuMAB-VEGF, and CAS Registry Number 216974-75-3, among others) and closely related VEGF inhibitors;
AVE-8062 (Ajinomoto Co. and Sanofi-aventis) (also called AC-7700 and combretastatin A4 analog, among others), and closely related VEGF inhibitors;
AZD-2171 (AstraZeneca) and closely related VEGF inhibitors;
Nexavar® (Bayer AG and Onyx) (also called CAS Registry Number 284461-73-0, BAY-43-9006, raf kinase inhibitor, sorafenib, sorafenib analogs, and IDDBCP150446, among others) and closely related VEGF inhibitors;
BMS-387032 (Sunesis and Bristol-Myers Squibb) (also called SNS-032 and CAS Registry Number 345627-80-7, among others) and closely related VEGF inhibitors;
CEP-7055 (Cephalon and Sanofi-aventis) (also called CEP-11981 and SSR-106462, among others) and closely related VEGF inhibitors;
CHIR-258 (Chiron) (also called CAS Registry Number 405169-16-6, GFKI, and GFKI-258, among others) and closely related VEGF inhibitors;
CP-547632 (OSI Pharmaceuticals and Pfizer) (also called CAS Registry Number 252003-65-9, among others) and closely related VEGF inhibitors such as, for instance, CP-564959;
E-7080 (Eisai Co.) (also called CAS Registry Number 417716-92-8 and ER-203492-00, among others) and closely related VEGF inhibitors;
786034 (GlaxoSmithKline) and closely related VEGF inhibitors;
GW-654652 (GlaxoSmithKline) and closely related indazolylpyrimidine Kdr inhibitors;
IMC-1C11 (ImClone) (also called DC-101 and c-p1C11, among others) and closely related VEGF inhibitors;
KRN-951 (Kirin Brewery Co.) and other closely related quinoline-urea VEGF inhibitors;
PKC-412 (Novartis) (also called CAS Registry Number 120685-11-2, benzoylstaurosporine, CGP-41251, midostaurin, and STI-412, among others) and closely related VEGF inhibitors;
PTK-787 (Novartis and Schering) (also called CAS Registry Numbers 212141-54-3 and 212142-18-2, PTK/ZK, PTK-787/ZK-222584, ZK-22584, VEGF-TKI, VEGF-RKI, PTK-787A, DE-00268, CGP-79787, CGP-79787D, vatalanib, ZK-222584, among others) and closely related anilinophthalazine derivative VEGF inhibitors;
SU11248 (Sugen and Pfizer) (also called SU-11248, SU-011248, SU-11248J, Sutent®, and sunitinib malate, among others) and closely related VEGF inhibitors;
SU-5416 (Sugen and Pfizer/Pharmacia) (also called CAS Registry Number 194413-58-6, semaxanib, 204005-46-9, among others) and closely related VEGF inhibitors;
SU-6668 (Sugen and Taiho) (also called CAS Registry Number 252916-29-3, SU-006668, and TSU-68, among others) and closely related VEGF inhibitors as described in, among others, WO-09948868, WO-09961422, and WO-00038519, particularly in parts pertaining to SU-6668 and closely related VEGF inhibitors, their structures and properties, and methods for making and using them;
VEGF Trap (Regeneron and Sanofi-aventis) (also called AVE-0005 and Systemic VEGF Trap, among others) and closely related VEGF inhibitors as described in, among others, WO-2004110490, particularly in parts pertaining to VEGF Trap and closely related VEGF inhibitors, their structures and properties, and methods for making and using them;
Thalidomide (Celgene) (also called CAS Registry Number 50-35-1, Synovir, Thalidomide Pharmion, and Thalomid, among others) and closely related VEGF inhibitors;
XL-647 (Exelixis) (also called EXEL-7647, among others) and closely related VEGF inhibitors;
XL-999 (Exelixis) (also called EXEL-0999, among others) and closely related VEGF inhibitors;
XL-880 (Exelixis) (also called EXEL-2880, among others) and closely related VEGF inhibitors;
ZD-6474 (AstraZeneca) (also called CAS Registry Number 443913-73-3, Zactima, and AZD-6474, among others) and closely related anilinoquinazoline VEGF inhibitors; and
ZK-304709 (Schering) (also called CDK inhibitors (indirubin derivatives), ZK-CDK, MTGI, and multi-target tumor growth inhibitor, among others) and other closely related compounds including the indirubin derivative VEGF inhibitors described in WO-00234717, WO-02074742, WO-02100401, WO-00244148, WO-02096888, WO-03029223, WO-02092079, and WO-02094814, particularly in parts pertinent to these and closely related VEGF inhibitors, their structures and properties, and methods for making and using them.

Also among VEGF inhibitors are: Pazopanib, CDP791, Enzastaurin, BIBF 1120, BAY 573952, BAY 734506, XL 184, IMC-1121B, CEP 701, SU 014813, SU 10944, SU 12662, OSI-930, and BMS 582664, and closely related VEGF inhibitors.

In addition to the foregoing inhibitors that act directly on VEGF or VEGFR, the following inhibitors have anti-angiogenic properties:
ZD-6126 (AstraZeneca and Angiogene) (also called CAS Registry Number 219923-05-4, N-acetylcolchinol phosphate, ANG-453, AZD-6126, ZD-6126 derivatives and ZM-445526, among others) and closely related VEGF inhibitors such as other inhibitors in the ANG-400 series;
Imatinib (Novartis) (also called CAS Registry Numbers 152459-95-5 and 220127-57-1, Glivec, Gleevec, STI-571, and CGP-57148, among others) and closely related VEGF inhibitors;
RAD-001 (Novartis) (also called CAS Registry Number 159351-69-6, RAD-001, SDZ-RAD, Certican, and everolimus, among others) and closely related VEGF inhibitors; and
BMS-354825 (Bristol-Myers Squibb) (also called CAS Registry Number 302962-49-8, Src/Abl kinase inhibitor, and dasatinib, among others) and closely related VEGF inhibitors.

Also useful in the invention in this are regard are Volociximab, CCI-779, 17-AAG, DMXAA, CI-1040, and CI-1033.

Among the VEGF inhibitors preferred in the invention are the following: (a) a compound described in US2003/0125339 or US. Pat. No. 6995162, particularly in parts disclosing VEGF inhibitors; (b) a substituted alkylamine derivative described in US2003/0125339 or US2003/0225106 or US. Pat. No. 6995162 or US. Pat. No. 6878714, particularly in parts disclosing VEGF inhibitors; (c) a non-naturally occurring humanized monoclonal antibody that binds to VEGF; (d) a substituted omega-carboxyaryl diphenyl urea or derivative thereof as described in WO00/42012, WO00/41698, US2005/0038080A1, US2003/0125359A1, US2002/0165394A1, US2001/003447A1, US2001/0016659A1, and US2002/013774A1, particularly in parts disclosing the foregoing VEGF inhibitors; (e) an anilinophthalazine or derivative thereof that binds to and inhibits the activity of multiple receptor tyrosine kinases including binding to the protein kinase domain and inhibition of VEGFR1 and VEGFR2; (f) (5-[5-fluoro-2-oxo-1,2- dihydroindol-(3Z)-ylidenemethyl]-2, 4-dimethyl-1*H-*pyrrole-3-carboxylic acid [2-diethylaminoethyl]amide) or derivatives thereof that are VEGF inhibitors; and (g) VEGF inhibitors as described in US2006/0241115, including those of Formula IV therein.

Among VEGF inhibitors are the following: (a) 4TBPPAPC or a closely related compound described in US2003/0125339 or US. Pat. No. 6995162, particularly in parts disclosing 4TBPPAPC and closely related VEGF inhibitors; (b) AMG 706 or a closely related substituted alkylamine derivative described in US2003/0125339 or US2003/0225106 or US. Pat. No. 6995162 or US. Pat. No. 6878714, particularly in parts disclosing AMG 706 and these closely related VEGF inhibitors; (c) Avastin™ or a closely related non-naturally occurring humanized monoclonal antibody that binds to VEGF, is a VEGF inhibitor, and is at least 90% identical in sequence to Avastin™; (d) Nexavar® or a closely related substituted omega-carboxyaryl diphenyl urea or derivative thereof described in WO00/42012, WO00/41698, US2005/0038080A1, US2003/0125359A1, US2002/0165394A1, US2001/003447A1, US2001/0016659A1, and US2002/013774A1, particularly in parts disclosing these VEGF inhibitors; (e) PTK/ZK or a closely related anilinophthalazine or derivative thereof that binds to and inhibits the activity of multiple receptor tyrosine kinases including binding to the protein kinase domain and inhibition of VEGFR1 and VEGFR2; (f) Sutent® or a closely related derivative of (5-[5-fluoro-2-oxo-1,2- dihydroindol-(3Z)-ylidenemethyl]-2, 4-dimethyl-1*H*-pyrrole-3-carboxylic acid [2-diethylaminoethyl]amide) that is a VEGF inhibitor; and (g) VEGF inhibitors as described in US2006/0241115, including those of Formula IV therein.

Among VEGF inhibitors are the following: (a) 4TBPPAPC, as described in US2003/0125339 or US. Pat. No. 6995162, particularly in parts disclosing 4TBPPAPC; (b) AMG 706, as described in US2003/0125339 or US. Pat. No. 6995162 or US. Pat. No. 6878714, particularly in parts disclosing AMG 706; (c) Avastin™; (d) Nexavar®, as described in WO00/42012, WO00/41698, US2005/0038080A1, US2003/0125359A1, US2002/0165394A1, US2001/003447A1, US2001/0016659A1, and US2002/013774A1, particularly in parts disclosing Nexavar®; (e) PTK/ZK; (f) Sutent®, and (g) VEGF inhibitors of Formula IV as described in US2006/0241115.

In this regard, VEGF inhibitors are further described below, among these AMG 706 of the invention.

### (1) AMG 706

AMG 706 is a multi-kinase inhibitor that interferes with the Kit, PDGF, and VEGF-signalling pathways, as described in US2003/0125339, or US. Pat. No. 6995162 or US. Pat. No. 6878714, particularly in parts pertinent AMG 706, its structure and properties, methods for making and using it, and other related compounds. It has a name of N-(2,3-dihydro-3,3-dimethyl-1H-indol-6-yl)-2-[(4-pyridinylmethyl)amino]-3-pyridinecarboxamide (I). As used herein the term AMG 706 includes pharmaceutically acceptable salts, in particular, the diphosphate salt, except as otherwise provided herein. AMG 706 is also known as motesanib diphosphate.

### (2) 4TBPPAPC

4TBPPAPC is a multi-kinase inhibitor that interferes with the Kit, PDGR, and VEGF-signalling pathways. Its name is N-(4-(1,1 1-dimethylethyl)phenyl)-2-((4-pyridinylmethy)amino-3-pyridinecarboxamide, as described in US Patent Application US2003/0125339, particularly in parts pertinent to 4TBPPAPC, its structure and properties, methods for making and using it, and other related compounds. 4TBPPAPC also is named N-[4-(tert-Butyl)phenyl]{2-[(4-pyridylmethy-1)amino](3-pyridyl)}carboxmide, as set forth in paragraph [0649] of the foregoing patent application.

### (3) Avastin™

Avastin™" is a recombinant humanized monoclonal antibody that binds directly to VEGF, and is sold by Genentech. It is also known as bevacizumab.

### (4) Nexavar®

Nexavar® (also known as BAY 43-9006 and sorafenib) is a substituted omega carboxy diphenyl urea that inhibits RAF-1 activation thereby decreasing RAF-1 dependent phosphorylation of MEK-1 and ERK-1, as described in US Patent Application No. 2003/0125359A1, WO03/047523A2, and Wilhelm et al., Current Pharmaceutical Design, vol. 8, pp. 2255-2257 (2002), particularly in parts pertinent to Nexavar®, its structure and properties, methods for making and using it, and other related molecules. Its chemical name is 4-(4-{3-[4-Chloro-3-(trifluoromethyl)phenyl]ureido}phenoxy)-N²-methylpyridine-2-carboxamide. A variety of derivatives of this compound have been produced. Among these are fluorinated derivatives described in US Patent Application 2005/0038080A1 and WO2005/009961A2, particularly as to these and other pharmaceutically active diphenyl urea compounds.

### (5) PTK/ZK

PTK787, also known as vatalanib, is a multi-VEGF receptor tyrosine kinase inhibitor that is said to block tumor angiogenesis and lymphangiogenesis. Its chemical structure is N-(4-chlorophenyl)-4-(pyridin-4-ylmethyl)phthalazin-1-amine. It also is known as PTK787/ZK, PTK787/ZK222584, and PTK/ZK. See Thomas, A., et al., J. of Clin. Oncology, 23(18): 4162-4171 (2005); US Patent Application 20050118600A1.

### (6) Sutent®

Sutent® is a small molecule receptor tyrosine kinase inhibitor with the following chemical structure (5-[5-fluoro-2-oxo-1,2- dihydroindol-(3Z)-ylidenemethyl]-2, 4-dimethyl-1*H-*pyrrole-3-carboxylic acid [2-diethylaminoethyl]amide). Sutent® is also known as sunitinib malate and SU11248, and is reported to have anti-angiogenic and anti-tumor activities. See Mendel, D., et al., Clinical Cancer Research, 9:327-337 (2003); Schlessinger, J., The Scientist, 19(7):17 (2005).

### Combinations of Ang2 and VEGF Inhibitors

The invention described relates to a combination of
(a) 2xCon4(C), and
(b) AMG 706 and pharmaceutically acceptable salts thereof; for use in treating a solid tumour in a subject. In particular in this regard, the invention relates to the use in combination of the Ang2 inhibitor described in the foregoing section thereon and elsewhere herein in combination with the VEGF inhibitor described in the foregoing section thereon and elsewhere herein.

In this regard, in the Ang2 inhibitor is 2xCon4(C) and the VEGF inhibitor is AMG 706.

### Indications and Diseases

A variety of disorders and diseases and the like can be treated in accordance with various aspects and embodiments of the invention. In particular, in accordance with certain of its preferred aspects and embodiments, the compositions, methods and other features of the invention are used to treat diseases that involve a deleterious effect of or on angiogenesis and/or vascularization, including those that require angiogenesis and/or vascularization, that are solid tumours. Compositions, methods, kits and the like, among other things, useful to treat, for instance, cancers, and other hyperproliferative conditions, such as hyperplasia, psoriasis, contact dermatitis, immunological disorders, and infertility, some of which are mentioned above, may be envisaged.

The invention further is useful in these regards and others, by way of providing compositions, as described herein for inhibiting solid tumour growth, including inhibiting processes of cellular proliferation, invasiveness, and metastasis in a subject, including human patients.

Solid tumours treatable by methods of the present invention include those that occur in mammals, including those that occur in humans and other primates, as well as pet or companion animals such as dogs and cats, laboratory animals such as rats, mice and rabbits, and farm animals, such as horses, pigs, sheep, and cattle. In certain of the particularly preferred aspects of the invention and preferred embodiments thereof, humans are highly preferred, particularly, human patients suffering from a variety of conditions, such as those particularly set forth herein.

Tumors or neoplasms include growths of tissue cells in which the multiplication of the cells is uncontrolled and progressive. Some such growths are benign, but others are termed malignant and may lead to death. Malignant neoplasms or cancers are distinguished from benign growths in that, in addition to exhibiting aggressive cellular proliferation, they may invade surrounding tissues and metastasize. Moreover, malignant neoplasms are characterized in that they show a greater loss of differentiation (greater dedifferentiation), and of their organization relative to one another and their surrounding tissues. This property is also called "anaplasia."

Neoplasm and cancers which are solid tumours whose invasiveness or metastasis is associated with Ang-2 expression or activity and with VEGF expression or activity are particularly among the diseases to which the invention may be advantageously applied.

Neoplasms treatable by the present invention are those which are solid tumors, *i.e.,* carcinomas and sarcomas. Carcinomas include those malignant neoplasms derived from epithelial cells that infiltrate (invade) the surrounding tissues and give rise to metastases. Adenocarcinomas are carcinomas derived from glandular tissue, or which form recognizable glandular structures. Another broad category of cancers to which the invention may be advantageously applied includes sarcomas, which are tumors whose cells are embedded in a fibrillar or homogeneous substance like embryonic connective tissue.

The types of cancer and tumor cells amenable to treatment according to the invention include, without limitation, cells of: ACTH-producing tumors, cancer of the adrenal cortex, bladder cancer, brain cancer, breast cancer, cervical cancer, colorectal cancer, endometrial cancer, esophageal cancer, Ewing's sarcoma, gallbladder cancer, head and neck cancer, Hodgkin's lymphoma, Kaposi's sarcoma, kidney cancer, liver cancer, lung cancer (small and non-small cell), malignant peritoneal effusion, malignant pleural effusion, melanoma, neuroblastoma, glioma, osteosarcoma, ovarian cancer, ovarian (germ cell) cancer, pancreatic cancer, penile cancer, prostate cancer, retinoblastoma, skin cancer, soft tissue sarcoma, squamous cell carcinomas, stomach cancer, testicular cancer, thyroid cancer, trophoblastic neoplasms, uterine cancer, vaginal cancer, cancer of the vulva, and Wilms' tumor.

The present invention thus provides compositions and methods useful for the treatment of a wide variety of cancers, which are solid tumors. Types of cancer that may be treated include, but are not limited to: adenocarcinoma of the breast, prostate, and colon; all forms of bronchogenic carcinoma of the lung; myeloid; melanoma; hepatoma; neuroblastoma; papilloma; apudoma; choristoma; branchioma; malignant carcinoid syndrome; carcinoid heart disease; carcinoma (*e.g.,* Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell); histiocytic disorders; histiocytosis malignant; Hodgkin's disease; immunoproliferative small lung cell carcinoma; reticuloendotheliosis; melanoma; chondroblastoma; chondroma; chondrosarcoma; fibroma; fibrosarcoma; giant cell tumors; lipoma; liposarcoma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; to phoblastic tumor.

Further, the following types of cancers may also be treated: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; Sertoli cell tumor; theca cell tumor; leiomyoma; leiomyosarcoma; myoblastoma; myoma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin; angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

### Routes of Administration

Inhibitors in accordance with the invention, in various embodiments, may be administered by a variety of suitable routes, well known to those skilled in the art of administering therapeutics to a subject. In embodiments of the invention in this regard, one or more inhibitors, as described elsewhere herein, are administered via the alimentary canal. In other embodiments one or more inhibitors as described elsewhere herein are administered parenterally. In various embodiments one or more inhibitors may be administered via the alimentary canal in conjunction with one or more other inhibitors administered parenterally.

Such routes in a variety of embodiments include but are not limited to administration of the compositions orally, ocularly, mucosally, topically, rectally, pulmonarily, such as by inhalation spray, and epicutaneously. The following parenteral routes of administration also are useful in various embodiments of the invention: administration by intravenous, intraarterial, intracardiac, intraspinal, intrathecal, intraosseous, intraarticular, intrasynovial, intracutaneous, intradermal, subcutaneous, peritoneal, and/or intramuscular injection. In some embodiments intravenous, intraarterial, intracutaneous, intradermal, subcutaneous and/or intramuscular injection are used. In some embodiments intravenous, intraarterial, intracutaneous, subcutaneous, and/or intramuscular injection are used.

In certain embodiments of the invention the compositions are administered locally, for instance by intraocular injection to treat ocular neovascularization, retinopathy, or age-related macular degeneration.

### Doses

The amount of compounds which are administered and the dosage regimen for treating a disease condition with the compounds and/or compositions of this invention depend on a variety of factors, including the age, weight, sex, and medical condition of the subject, the type of disease, the severity of the disease, the route and frequency of administration, and the particular compound employed. Thus, the dosage regimen may vary widely.

Appropriate dosage of VEGF and Ang2 inhibitors to be used in combination in accordance with the invention may be determined by using well-known techniques employed more generally in pharmaceutical and related arts to determine the dosages of single agents and/or combinations of other agents for therapeutic uses.

In certain embodiments in this regard the dose of one or both inhibitors for use in a combination in accordance with the invention may be based partly or wholly on the optimum dose established for either one or for both of the inhibitors used alone. The optimum dose for each agent may be the dose recommended or set by the US Food and Drug Administration (particularly as to formulations for use in the US) and/or by other authorities having jurisdiction over such matters outside the US (particularly as to formulations for use outside the US), for the use of the agent by itself or in other combination formulations.

It is to be understood that the optimum dose in some cases is the dose that achieves the maximum therapeutic effect, but in other cases may be higher or lower. For instance, administration of any agent at the dose that achieves maximum therapeutic effect may, at the same time, produce unacceptable side effects such as toxicity, so that the optimum dose is lower than that which has the maximum therapeutic effect.

Accordingly, in some combinations in accordance with the invention, one or both agents will be present at the dose that achieves the maximum therapeutic effect when used alone and/or when used in the combination. In other combinations in accordance with the invention one or the other or both agents will be present in the amount that achieves the optimum therapeutic effect when the agent is used by itself. In still other embodiments one or both agents will be present at a dose that achieves the optimum therapeutic effect for that agent when used in the combination, representing a balance between therapeutic effects and undesirable side effects such as toxicity.

It is to be further appreciated that among advantages of combination therapies in accordance with the invention, is not only that in some embodiments the combination works better than either single agent by itself (at the same doses) but also that the combination provides at least the same therapeutic effect or better than either agent by itself, using a dose of one or both agents less than that required to achieve the same effect when either agent is used by itself. In embodiments such reduced dosages result in fewer side effects and/or decreased toxicity.

The same considerations apply when more than two agents are used in combination with one another, such as when three, four, five or more agents are used in combination.

In accordance with the foregoing, the doses of agents to be used in combination in embodiments are the doses recommended, set, or prescribed by the US Food and Drug Administration or by an agency of similar authority outside the US. In embodiments, furthermore, the dose of an agent used in a combination therapy according to the invention also may be less, or more, than the aforementioned recommended, set, or prescribed doses, such as, in particular, when the therapeutic profile of the agent in the combination therapy is optimal at such lower or higher dose. In embodiments in this regard, the dose of one or more of the agents in the combination therapy is 10%, 25%, 50%, 75%, 85%, 95%, 105%, 110%, 125% or more of the doses recommended, set, or prescribed by such an agency for use of the agent alone, particularly as to use of the agent alone for the same indication.

In the case of agents that are administered parenterally, such as by IV injection, in embodiments of the invention the dose of the agent for combination therapy is 0.05 mg/kg to 100 mg/kg. In embodiments it is 0.01 to 50 mg/kg. In embodiments it is 0.02 to 40 mg/kg. In embodiments it is 0.3 to 30 mg/kg. In embodiments it is 0.5 to 25 mg/kg. In embodiments it is 1.0 to 15 mg/kg. Further in this regard, in embodiments the dose is any one or more of 0.05, 0.1, 0.2, 0.3, 0.5, 1.0, 2.0, 3.0, 5.0, 10, 15, 20, 25, 30, 40, 50, 60, 70, 80, 100 mg/kg or more. In this regard the doses may be administered at any interval, including but not limited to more than once a day, once a day, every two, three, four, five, six or more days, once a week, every one, two, three, or four weeks, or at longer intervals.

For example, in embodiments in this regard, doses of 2xCon4(C) in combination therapies in accordance with the invention, in addition to the above, are for 0.1 to 50 mg/kg IV once a week. In embodiments the doses are 0.3 mg/kg to 30 mg/kg IV once a week. In embodiments the dose is 3 mg/kg IV once a week. In embodiments it is 5 mg/kg IV once a week. In embodiments it is 10 mg/ml IV once a week. In embodiments it is 15 mg/kg IV once a week.

Much the same considerations as those described immediately above apply as well to the dose of an agent for oral administration in a combination therapy in accordance with the invention. As noted elsewhere herein, for oral administration the pharmaceutical composition may be in the form of, for example, a tablet, capsule, suspension, or liquid. The pharmaceutical composition is preferably made in the form of a dosage unit containing a particular amount of the active ingredient. Examples of such dosage units are tablets or capsules. General principles and methods for formulating agents for oral administration are well know to those skilled in the art.

Formulations of agents for oral administration in combination therapy in accordance with the invention in embodiments thereof contain the active ingredient in an amount from less than 1 to 2,000 mg, or more. In embodiments the amount is from 1 to 1,000, 10 to 1,000, 50 to 500, 75 to 750 mg, among others. For example, without limitation, in embodiments the amount is 1, 10, 25, 50, 75, 100, 200, 250, 300, 400, 500, 600, 700, 800, 1,000, or 2,000 mg per dosage form.

In embodiments the agent is administered in combination therapy in accordance with the invention in a doses of 0.01 or less to 2,000 mg/kg or more. In embodiments the dose is 1 to 1,000 mg/kg. In embodiments it is 15 to 750 mg/kg. In embodiments it is 25 to 700 mg/kg. In embodiments it is 50 to 500 mg/kg. For example, in embodiments it is 1, 10, 25, 50, 75, 100, 200, 250, 300, 400, 500, 600, 700, 800, 1,000, or 2,000 mg/kg.

As for other forms of administration, doses for oral administration may be administered one, two, three, four, five, six, seven or more times per day, or at intervals of more than a day, such as every two, three, four, five, six, or seven days, or at longer intervals.

Oral administration of an agent in combination therapy in accordance with the invention as noted above will vary with many factors, including the exact agent, and the indication and the immediate condition of the patient, to name just a few. By way of illustration only, keeping in mind this variability, in embodiments in this regard, the agent is administered orally in a dosage form containing, 1, 10, 25, 50, 75, 100, 200, 250, 300, 400, 500, 600, 700, 800, 1, 000, or 2,000 mg each. In embodiments in this regard, the agent is orally administered in one or more of the foregoing amounts one, two, three, four or more times per day, or at lesser intervals. In embodiments in this regard, one, two, three, or four dosage units are administered at the same time.

In embodiments including embodiments in which administration is by parenteral, oral, or other means, dosing intervals are varied, such as between two, three or more frequencies of administration, or by skipping or adding administrations at set intervals or in accordance with patient response. In embodiments doses are altered between responses, such as regular variation between two, three, four or more dosing regimes, or in accordance with patient response.

The foregoing considerations apply to both Ang2 and VEGF inhibitors used in combination therapy in accordance with the invention, among others.

Dosing regimens are discussed in further detail below.

### Dosing Regimens

Compositions can be administered in dosages and by techniques well known to those skilled in the medical and veterinary arts taking into consideration such factors as the age, sex, weight, and condition of the particular patient, and the formulation that will be administered (e.g., solid vs. liquid). Doses for humans or other mammals can be determined without undue experimentation by the skilled artisan, from this disclosure, the documents cited herein, and the knowledge in the art.

In accordance with various embodiments, proper dosages and dosing plans will depend on numerous factors, and may vary in different circumstances. The parameters that will determine the optimal dosage plans to be administered typically will include some or all of the following: the disease being treated and its stage; the species of the subject, their health, gender, age, weight, and metabolic rate; other therapies being administered; and expected potential complications from the subject's history or genotype.

The optimal dosing plan in a given situation also will take into consideration the way in which the compositions are formulated, the way they are administered, their distribution route following administration, and the rate at which they will be cleared both from sites of action and from the subject's body. Finally, the determination of optimal dosing necessarily will provide an effective dose that is neither below the threshold of maximal beneficial effect nor above the threshold where the deleterious effects associated with the dose of the active agents outweighs the advantages of the increased dose.

It will be appreciated that a "dose" may be delivered all at once, fractionally, or continuously over a period of time. The entire dose also may be delivered to a single location or spread fractionally over several locations. Furthermore, doses may remain the same over a treatment, or they may vary.

In various embodiments, compositions of the invention are administered in an initial dose, and thereafter maintained by further administrations. A composition of the invention in some embodiments is administered by one method initially, and thereafter administered by the same method or by one or more different methods. The dosages of on-going administrations may be adjusted to maintain at certain values the levels of the active agents in the subject. In some embodiments the compositions are administered initially and/or to maintain their level in the subject by intravenous injection. In a variety of embodiments, other forms of administration are used, dependent upon the patient's condition and other factors, discussed elsewhere herein.

Suitable regimens for initial administration and further doses for sequential administrations may all be the same or may be variable. Appropriate regimens can be ascertained by the skilled artisan, from this disclosure, the documents cited herein, and the knowledge in the art. The dose, frequency, and duration of treatment will depend on many factors, including the nature of the disease, the subject, and other therapies that may be administered.

Accordingly, a wide variety of regimens may be used to administer the compositions. In some embodiments they are administered to a subject in one dose. In others they are administered to a subject in a series of two or more doses in succession. In some other embodiments where they are administered in a single dose, in two doses, and/or more than two doses, the doses may be the same or different, and they may be administered with equal or with unequal intervals between them.

Compositions of the invention may be administered in many frequencies over a wide range of times, including any suitable frequency and range of times that delivers a treatment-effective dose. Doses may be continuously delivered, administered every few hours, one or more times a day, every day, every other day or several times a week, or less frequently. In some embodiments they are administered over periods of one, two, three, four, five, six, seven, eight, nine, ten, eleven, twelve, thirteen, fourteen, or more days. In some embodiments they are administered over periods of one, two, three, four, five, six, seven, eight, nine, ten, eleven, twelve, or more months. In a variety of embodiments they are administered for a period of one, two, three, four, five, six, seven, eight, nine, ten, or more years. Generally lengths of treatment will be proportional to the length of the disease process, the effectiveness of the therapies being applied, and the condition and response of the subject being treated.

In various embodiments of the invention, inhibitors are administered one to four times per day, in equal amounts, at equal intervals.

### Formulations

Pharmaceutical compositions in accordance with various embodiments of the invention may comprise one or more non-toxic, pharmaceutically-acceptable carriers and/or diluents and/or adjuvants (collectively referred to herein as "carrier" materials) and, if desired, other active ingredients. The pharmaceutically active compounds of this invention can be processed in accordance with conventional methods of pharmacy to produce medicinal agents for administration to human patients and to other subjects.

Formulations for administration of biologics such as proteins, including antibodies, are well known in the art. For example, see US 6,171,586; WO 2005/044854; US 6,288,030; US 6,267,958; WO 2004/055164; US 4,597,966; US 2003/0138417; US 6,252,055; US 5,608,038; US 6,875,432; US 2004/0197324; WO 02/096457; US 5,945,098; US 5,237,054; US 6,485,932; US 6,821,515; US 5,792,838; US 5,654,403; US 5,908,826; EP 0 804 163; and WO 2005/063291, in particular in parts pertinent to formulation, stabilization, and delivery of protein-based drugs. As described in the foregoing and elsewhere, among preferred buffering agents for proteins known in the art are acetate, succinate, gluconate, glutamate, glutamic acid, histidine, citrate, aspartic acid, and other organic acid buffers.

For oral administration, the pharmaceutical composition may be in the form of, for example, a tablet, capsule, suspension, or liquid. The pharmaceutical composition is preferably made in the form of a dosage unit containing a particular amount of the active ingredient. Examples of such dosage units are tablets or capsules.

For therapeutic purposes, the active compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide; sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e., Captisol), cosolvent solubilization (i.e., propylene glycol), or micellar solubilization (i.e., Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

For pulmonary administration, the pharmaceutical composition may be administered in the form of an aerosol or with an inhaler including dry powder aerosol.

The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

Pharmaceutical compositions in accordance with the foregoing and other aspects of the invention may contain formulation materials for modifying, maintaining or preserving, for example, the pH, osmolarity, viscosity, clarity, color, isotonicity, odor, sterility, stability, rate of dissolution or release, adsorption or penetration of the composition.

Suitable formulation materials include, but are not limited to, amino acids (such as glycine, glutamine, asparagine, arginine or lysine); antimicrobials; antioxidants (such as ascorbic acid, sodium sulfite or sodium hydrogen-sulfite); buffers (such as borate, bicarbonate, Tris-HCl, citrates, phosphates, other organic acids); bulking agents (such as mannitol or glycine), chelating agents [such as ethylenediamine tetraacetic acid (EDTA)]; complexing agents (such as caffeine, polyvinylpyrrolidone, beta-cyclodextrin or hydroxypropyl-beta-cyclodextrin); fillers; monosaccharides; disaccharides and other carbohydrates (such as glucose, mannose, or dextrins); proteins (such as serum albumin, gelatin or immunoglobulins); coloring; flavoring and diluting agents; emulsifying agents; hydrophilic polymers (such as polyvinylpyrrolidone); low molecular weight polypeptides; salt-forming counterions (such as sodium); preservatives (such as benzalkonium chloride, benzoic acid, salicylic acid, thimerosal, phenethyl alcohol, methylparaben, propylparaben, chlorhexidine, sorbic acid or hydrogen peroxide); solvents (such as glycerin, propylene glycol or polyethylene glycol); sugar alcohols (such as mannitol or sorbitol); suspending agents; surfactants or wetting agents (such as pluronics, PEG, sorbitan esters, polysorbates such as polysorbate 20, polysorbate 80, triton, tromethamine, lecithin, cholesterol, tyloxapal); stability enhancing agents (sucrose or sorbitol); tonicity enhancing agents (such as alkali metal halides (preferably sodium or potassium chloride, mannitol sorbitol); delivery vehicles; diluents; excipients and/or pharmaceutical adjuvants. (Remington's Pharmaceutical Sciences, 18th Edition, A.R. Gennaro, ed., Mack Publishing Company, 1990).

The optimal pharmaceutical composition will be determined by one skilled in the art depending upon, for example, the intended route of administration, delivery format, and desired dosage. See for example, Remington's Pharmaceutical Sciences, supra. Such compositions may influence the physical state, stability, rate of in vivo release, and rate of in vivo clearance of the specific binding agent.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of pH 7.0-8.5, or acetate buffer of pH 4.0-5.5, which may further include sorbitol or a suitable substitute therefore. In one embodiment of the present invention, binding agent compositions may be prepared for storage by mixing the selected composition having the desired degree of purity with optional formulation agents (Remington's Pharmaceutical Sciences, supra) in the form of a lyophilized cake or an aqueous solution. Further, the binding agent product may be formulated as a lyophilizate using appropriate excipients such as sucrose.

The pharmaceutical compositions can be selected for parenteral delivery. Alternatively, the compositions may be selected for inhalation or for enteral delivery such as orally, aurally, opthalmically, rectally, or vaginally. The preparation of such pharmaceutically acceptable compositions is within the skill of the art.

The formulation components are present in concentrations appropriate to the site of administration. For example, buffers are used to maintain the composition at physiological pH or at slightly lower pH, typically within a pH range of from 5 to 8.

When parenteral administration is contemplated, the therapeutic compositions for use in this invention may be in the form of a pyrogen-free, parenterally acceptable aqueous solution comprising the desired specific binding agent in a pharmaceutically acceptable vehicle. A particularly suitable vehicle for parenteral injection is sterile distilled water in which a binding agent is formulated as a sterile, isotonic solution, properly preserved.

Yet another preparation can involve the formulation of the desired molecule with an agent, such as injectable microspheres, bio-erodible particles, polymeric compounds (polylactic acid, polyglycolic acid), beads, or liposomes, that provides for the controlled or sustained release of the product which may then be delivered via a depot injection. Hyaluronic acid may also be used, and this may have the effect of promoting sustained duration in the circulation. Other suitable means for the introduction of the desired molecule include implantable drug delivery devices.

In another aspect, pharmaceutical formulations suitable for parenteral administration may be formulated in aqueous solutions, preferably in physiologically compatible buffers such as Hanks' solution, ringer's solution, or physiologically buffered saline. Aqueous injection suspensions may contain substances that increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions.

A pharmaceutical composition in accordance with certain embodiments of the invention may be suitable for inhalation. For example, a binding agent may be formulated as a dry powder for inhalation. Polypeptide or nucleic acid molecule inhalation solutions may also be formulated with a propellant for aerosol delivery. In yet another embodiment, solutions may be nebulized. Pulmonary administration is further described in PCT Application No. PCT/US94/001875, which describes pulmonary delivery of chemically modified proteins.

It is also contemplated that certain formulations may be administered orally. In one embodiment of the present invention, binding agent molecules that are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the binding agent molecule. Diluents, flavorings, low melting point waxes, vegetable oils, lubricants, suspending agents, tablet disintegrating agents, and binders may also be employed.

Pharmaceutical compositions for oral administration can also be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combining active compounds with solid excipient and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol, and sorbitol; starch from corn, wheat, rice, potato, or other plants; cellulose, such as methyl cellulose, hydroxypropylmethylcellulose, or sodium carboxymethylcellulose; gums, including arabic and tragacanth; and proteins, such as gelatin and collagen. If desired, disintegrating or solubilizing agents may be added, such as the cross-linked polyvinyl pyrrolidone, agar, and alginic acid or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations that can be used orally also include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Another pharmaceutical composition may involve an effective quantity of binding agent in a mixture with non-toxic excipients that are suitable for the manufacture of tablets. By dissolving the tablets in sterile water, or other appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional pharmaceutical compositions will be evident to those skilled in the art, including formulations involving binding agent molecules in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, PCT/US93/00829 that describes controlled release of porous polymeric microparticles for the delivery of pharmaceutical compositions. Additional examples of sustained-release preparations include semipermeable polymer matrices in the form of shaped articles, e.g. films, or microcapsules. Sustained release matrices may include polyesters, hydrogels, polylactides (U.S. 3,773,919, EP 58,481), copolymers of L-glutamic acid and gamma ethyl-L-glutamate [Sidman et al., Biopolymers, 22:547-556 (1983)], poly (2-hydroxyethyl-methacrylate) [Langer et al., J. Biomed. Mater. Res., 15:167-277, (1981)] and [Langer et al., Chem. Tech., 12:98-105(1982)], ethylene vinyl acetate (Langer et al., supra) or poly-D(-)-3-hydroxybutyric acid (EP 133,988). Sustained-release compositions also include liposomes, which can be prepared by any of several methods known in the art. See e.g., Eppstein et al., Proc. Natl. Acad. Sci. (USA), 82:3688-3692 (1985); EP 36,676; EP 88,046; EP 143,949.

The pharmaceutical composition to be used for in vivo administration typically must be sterile. This may be accomplished by filtration through sterile filtration membranes. Where the composition is lyophilized, sterilization using this method may be conducted either prior to or following lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts of compounds may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, example of which are formic, acetic, adipic, butyric, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, ethanedisulfonic, benzenesulfonic, pantothenic, 2-hydroxyethanesulfonic, toluenesulfonic, sulfanilic, cyclohexylaminosulfonic, camphoric, camphorsulfonic, digluconic, cyclopentanepropionic, dodecylsulfonic, glucoheptanoic, glycerophosphonic, heptanoic, hexanoic, 2-hydroxy-ethanesulfonic, nicotinic, 2-naphthalenesulfonic, oxalic, palmoic, pectinic, persulfuric, 2-phenylpropionic, picric, pivalic propionic, succinic, tartaric, thiocyanic, mesylic, undecanoic, stearic, algenic, β-hydroxybutyric, salicylic, galactaric and galacturonic acid. Suitable pharmaceutically-acceptable base addition salts of compounds include metallic salts, such as salts made from aluminum, calcium, lithium, magnesium, potassium, sodium and zinc, or salts made from organic bases including primary, secondary and tertiary amines, substituted amines including cyclic amines, such as caffeine, arginine, diethylamine, N-ethyl piperidine, aistidine, glucamine, isopropylamine, lysine, morpholine, N-ethyl morpholine, piperazine, piperidine, triethylamine, trimethylamine. All of these salts may be prepared by conventional means from the corresponding compound of the invention by reacting, for example, the appropriate acid or base with the compound.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form (e.g., lyophilized) requiring reconstitution prior to administration.

### Kits

Kits comprising one or more Ang2 inhibitors and one or more VEGF inhibitors in accordance with the foregoing may be envisaged. The inhibitors may be disposed in the kits in one or more containers. Each such container may contain separately or in admixture one or more Ang2 inhibitors and one or more VEGF inhibitors in accordance with any of the foregoing. Typically, such kits are designed for medical use, and the inhibitors are comprised in pharmaceutically acceptable formulations. Among kits in this regard are those comprising 2xCon4(C) and AMG 706. Also in this regard are kits wherein the inhibitors are disposed in separate containers. Kits may be those that comprise integrally thereto or as one or more separate documents, information pertaining to the contents or the kit and the use of the inhibitors. Also kits may be those wherein the compositions are formulated for reconstitution in a diluent. Kits may further comprise one or more containers of sterile diluent. Kits wherein at least one of the inhibitors is disposed in vials under partial vacuum sealed by a septum and suitable for reconstitution to form a formulation effective for parental administration, may be envisaged.

Kits that provide single-dose packaging of one or more of the inhibitors, and kits that include those that provide single and multi-chambered pre-filled syringes (e.g., liquid syringes and lyosyringes) for administering one or more of the inhibitors, and kits in which the syringes are preloaded, may be envisaged.

The present invention is additionally described by way of the following illustrative, non-limiting examples.

### Examples

### COMPARATIVE EXAMPLE 1: Tek472/Fc and Avastin™

Xenografts of Colo205 cells injected into female CD1 Nu/Nu mice were treated with: (1) Avastin™ (group 1); (2) both Avastin™ and Tek472/Fc (group 2); (3) HuIgG1 and Fc control (group 3), and (4) Tek472/Fc (group 4), as described in greater detail below.

Tumors were induced by injecting Colo205 cells into forty CD1 Nu/Nu female mice, 6 to 8 weeks of age, weighing between 20 and 22 grams. All of the mice were obtained from Charles River Laboratories (Raleigh, NC, APR# 245658). Colo205 cells for injection were cultured in suspension at 37° C in McCoy5A medium (Gibco/BRL, Grand Island, NY) supplemented with 10% FBS (Hyclone, Logan, Utah). For injection, cells were harvested from culture and mixed with Matrigel™ (BD Bioscience, Bedford, MA, Cat#354234) to a concentration of 1 x 10⁷ cells/ml. 0.2 ml of the cell-Matrigel™ mixture containing 2 x 10⁶ Colo205 cells was injected into the right flank of each mouse subcutaneously. Mice then were randomly assigned to the control and treatment groups (summarized in Table 1A), marked, and weighed.

Twice each week, throughout the study, the length, height, and width of each tumor was measured, and the mice were weighed. Tumor volumes were calculated from the dimensional measurements (length times width times height) and uniformly expressed in mm³.

Before use, the Tek472/Fc, Fc, HuIgG1, and Avastin™ preparations were diluted in PBS at a concentration of 1 mg/ml (0.1%). 100 µl doses were administered ip or sc. Treatment amounts and routes of administration are set out in Table 1A for all of the groups.

**Table 1A**

| Group No. | No. of Mice | Treatment 1 | Frequency | Treatment 2 | Frequency |
|---|---|---|---|---|---|
| 1 | 10 | Avastin™ 100 µg ip | 2x/week | Fc 40 µg sc | 3x/week |
| 2 | 10 | Avasrin™ 100 µg ip | 2x/week | Tek472/Fc 40 µg sc | 3x/week |
| 3 | 10 | HuIgG1 100 µg ip | 2x/week | Fc 40µg sc | 3x/week |
| 4 | 10 | HuIgG1 100 µg ip | 2x/week | Tek472/Fc 40µg sc | 3x/week |

All treatments were started 21 days after cells were injected into the mice, when the tumor volumes were around 450 mm³.

A double blind procedure was used throughout the study, whereby the persons measuring the tumor volumes and administering the drugs were unaware of the treatment.

All results are expressed as the mean ± standard error of the mean ("SEM"). Data were statistically analyzed with factorial ANOVA for repeated measurements, using StatView software v5.0.1 (SAS Institute, Cary, NC). Scheffe's post hoc test was used to determine p values for repeated measurements. Comparison groups with p values of 0.05 or less were considered to be significantly different.

Results are graphically presented in Figures 1, which shows the tumor volumes (mean ± SEM) for each of the four groups over the course of the study. Treatment with Avastin™ alone (Group 1) or Tek472/Fc alone (Group 4) significantly inhibited tumor growth when compared to treatment with the control by itself (Group 3): p<0.0001 for Avastin™ and p=0.0175 for Tek472/Fc. Treatment with both Tek472/Fc and Avastin™ (Group 2) was significantly more effective than treatment with the control (Group 3): p<0.0001. It also was significantly more effective, p<0.0001, than treatment with Tek472/Fc alone (Group 1). However, treatment with Avastin™ by itself was as effective as treatment with both Tek472/Fc and Avastin™.

The mean weights of the animals for each group did not differ in a statistically significant manner over the course of the study.

In sum, as measured by tumor volume, each agent alone and the combination significantly inhibited tumor growth compared to the control. The combination group was statistically superior to treatment with Tek472/Fc alone (p<0.0001) but not Avastin™ alone.

### COMPARATIVE EXAMPLE 2: Anti-Ang2 (Ab 536) and Anti-VEGF

Xenografts of A431 cells injected into female CD1 Nu/Nu mice were treated with: (1) control human IgG1 (kappa); (2) anti-Ang2 antibody (Ab 536); (3) anti-VEGF antibody; and (4) both anti-VEGF antibody and anti-Ang2 antibody (Ab 536), as presented in greater detail in Tables 2A and 2B and in the discussion below.

**TABLE 2A**

| Group No. | No of Mice | Treatment |
|---|---|---|
| 1 | 10 | Control human IgG1 (Kappa) |
| 2 | 10 | Anti-Ang2 (Ab 536) |
| 3 | 10 | Anti-VEGF + Control IgG1 (kappa) |
| 4 | 10 | Anti-VEGF + Anti-Ang2 (Ab 536) |

**TABLE 2B**

| | Anti-Ang2 AB 536 | Human IgG1 Kappa | Anti-VEGF |
|---|---|---|---|
| Supplier | n/a | Sigma | R&D |
| Vehicle | PBS | PBS | PBS |
| Dose | First dose 140 µg, then 46.7µg each dose | First dose 140 µg, then 46.7µg each dose | First dose 15.5 µg, then 5.18 µg each dose |
| Route | IP | IP | IP |
| Schedule | 3x/week | 3x/week | 3x/week |
| Mice | CD1 Nu/Nu, Female, Charles River Laboratories | | |

The A431 cells were cultured in T-225 flasks. After 40 passages, cells for the study were grown until they were 80-85% confluent and then harvested using trypsin/EDTA. Harvested cells were suspended in RPMI 1740 medium at a concentration of 5 x 10⁷ cells/ml. More then 95% of the cells in the preparation used for this study were viable as determined by trypan blue exclusion.

Tumors were induced by injecting 1 x 10⁷ A431 cells suspended in 0.2 ml of serum free media subcutaneously into the right flank of 40 CD1 Nu/Nu female mice, 6 to 8 weeks of age, weighing between 20 and 22 grams.

Before use, the anti-Ang2, HuIgG1, and anti-VEGF antibodies were diluted in PBS. All treatments were initiated three days after the cells were injected, before the tumors were palpable.

To start treatment the mice in each group were injected with an initial dose that was three times higher than the subsequent doses in the study. The groups, the initial doses, and the continuing doses are set out in detail in Tables 2A and 2B.

Tumors were measured and mice were weighed throughout the study as described in Example 1.

Results are expressed and the data were analyzed as described in Example 1.

Data from the study are presented in Figures 2.

The graph in Figure 2 displays the mean tumor volume for each of group 1 (control), group 2 (anti-Ang2 antibody, Ab 536), group 3 (anti-VEGF antibody), and group 4 (both antibodies), over the course of the study. The vertical bars represent the ± SEM. The indicated p values refer to comparisons with the control (IgG 1) (group 1) based on the tumor volumes as a function of time using the repeated measure ANOVA with Scheffe's post hoc test.

Animal weights were measured over the course of the study. All of the groups maintained their weights, and by this measure, there was no evidence that any of the treatments were significantly deleterious to the animal's overall condition.

As shown in Figure 2, treatment with the anti-Ang2 (Ab 536) antibody alone (group 2) and the anti-VEGF antibody alone (group 3) inhibited growth of the xenografts significantly more than the control treatment (group 1): p=0.0012 and p=0.0004, respectively. Treatment with both antibodies together also effectuated a statistically significant inhibition of tumor growth, relative to the control, p<0.0001. However, treatment with both antibodies was not significantly more efficacious than treatment with either one by itself.

### EXAMPLE 3: 2xCon4(C) and AMG 706-HT29 Xenografts

Xenografts of HT29 cells injected into female athymic nude mice were treated with 2xCon4(C) alone, AMG 706 alone, or a combination of the two, as described below.

Tumors were induced by injecting HT29 cells subcutaneously into 90 female athymic nude mice, 6 to 8 weeks of age, weighing between 21 and 23 grams. All of the mice were obtained from Hirlan Sprague Dawley. HT29 cells for injection were cultured in suspension at 37° C in McCoy5A1 medium (GibcoBRL, Grand Island, NY) supplemented with 10% FBS (Hyclone, Logan, Utah). After 26 passages, cells for the study were grown until they were semi-confluent (approximately 40%) and then harvested using trypsin/EDTA. Cells were mixed with Matrigel™ (BD Bioscience, Bedford, MA, Cat#354234) in a ratio of 2:1 to a concentration of 1 x 10⁷ cells/ml. Greater than 95% of the cells were viable as determined by trypan blue exclusion. 0.2 ml of the cell-Matrigel™ mixture containing 2 x 10⁶ HT29 cells was injected into the right flank of each mouse subcutaneously.

Twenty-one days after injection, 40 of the 90 mice were selected for the study by computer using computer randomizing and sorting programs, and treatment was initiated.

Twice each week, throughout the study, the length, height, and width of each tumor was measured and the mice were weighed. Tumor volumes were calculated from the dimensional measurements length x width x height and uniformly expressed in mm³.

Before use, 2xCon4(C) and the Fc control were each diluted to working concentration (dose/0.1 ml) in PBS. AMG 706 was diluted to working concentration with Ora-Plus® adjusted with methanesulfonic acid to pH 2.0 (dose/0.2 ml). All of compounds were stored at 4°C.

The treatment regimen for each group of mice is set out in Table 3A. Treatment started on day 21 after tumor implantation. Groups were treated with: (1) vehicle and Fc control (group 1), (2) AMG 706 and Fc (group 3), (3) 2xCon4(C) plus vehicle (group 2), and (4) AMG 706 and 2xCon4(C) (group 4). 2xCon4(C) was given at 14 µg/dose, subcutaneously, twice per week (group 2). AMG 706 was given orally at the dose of 75 mg/kg QD (group 3). The same doses and dosing schedule were used for the combination treatment of 2xCon4(C) and AMG 706 (group 4). Vehicle served as the control for AMG 706. Fc served as the control for 2xCon4(C). Group 1 was treated only with the two controls.

**TABLE 3A**

| Group No. | Mouse Nos. | Treatment 1 | Treatment 2 |
|---|---|---|---|
| 1 | 1-10 | Vehicle, po, QD | Fc, 14 µg, sc, 2x/week |
| 2 | 11-20 | Vehicle, po, QD | 2xCon4(C), 14 µg, sc, 2x/week |
| 3 | 21-30 | AMG 706, 75 mg/kg, po, QD | Fc, 14 µg, sc, 2x/week |
| 4 | 31-40 | AMG 706, 75 mg/kg, po, QD | 2xCon4(C), 14 µg, sc, 2x/week |

Results are expressed and the data were analyzed as described in Example 1.

Data are presented in Figure 3.

The graph in Figure 3 displays the mean tumor volume for each of group 1 (control), group 2 (2xCon4(C)), group 3 (AMG 706), and group 4 (2xCon4(C) and AMG 706) over the course of the study. The vertical bars represent ± SEM. As indicated by a thick vertical arrow, treatment began on day 21 post-implantation and ended on day 41.

Animal weights were measured over the course of the study. The body weights remained relatively stable for all groups. By this measure, none of the treatments were substantially deleterious or toxic.

As measured by tumor volume and depicted in Figure 3, all three treatment groups, compared to the control, had a statistically significant inhibitory effect on tumor growth: p<0.0001 (groups 3 and 4), and p=0.0078 (group 2). In addition, as measured by tumor volume and depicted in Figure 3, combination treatment (group 4) was significantly more effective than treatment with either 2xCon4(C) by itself (group 2), pH.0385, or AMG 706 by itself (group 3), p<0.0001.

### COMPARATIVE EXAMPLE 4: 2xCon4(C) and 4TBPPAPC

As described below, xenografts of Colo205 cells injected into female CD1 Nu/Nu mice were treated with: (1) control vehicle, (2) 4TBPPAPC (low dose), (3) 2xCon4(C) (low dose), (4) 4TBPPAPC (high dose), (5) 2xCon4(C) (high dose), (6) 4TBPPAPC and 2xCon4(C) (both low doses), (7) 4TBPPAPC (low dose) and 2xCon4(C) (high dose), (8) 4TBPPAPC (high dose) and 2xCon4(C) (low dose), and (9) 4TBPPAPC and 2xCon4(C) (both high doses).

Tumors were induced by injecting Colo205 cells subcutaneously into 120 female CD1 Nu/Nu mice, 8-10 weeks old, weighing between 22 and 25 grams. All of the mice were obtained from Charles River Laboratories (Raleigh, NC). Colo205 cells (passage #Xp9) for injection were cultured in T-225 culture flasks until they were 70% confluent. Cells were harvested for injection using trypsin/EDTA. The cells were >95% viable by trypan blue exclusion. The cells were resuspended in serum free medium containing 1/3 volume of Matrigel™ to 1 x 10⁷ cells/ml. 0.2 ml of the cell-Matrigel™ mixture containing 2 x 10⁶ Colo205 cells was injected subcutaneously into the right flank of each mouse.

Twenty-one days after injection, when the tumor volumes were approximately 500 mm³, 90 of the 120 mice were selected for the study by computerized randomizing and sorting, and treatment was initiated the same day on these mice. There were 10 mice in each group. Twice each week throughout the study, the length, height, and width of each tumor was measured, and the mice were weighed beginning on day 12 post-implantation. Tumor volumes were calculated from the dimensional measurements as length x width x height, and uniformly expressed in mm³.

Before use, 2xCon4(C) was diluted to a working concentration (dose/0.1 ml) in PBS. 4TBPPAPC was diluted to a working concentration (dose/0.2 ml) in Ora-Plus® adjusted with methanesulfonic acid to pH 2.0.

Treatment started on day 21 post-tumor implantation. Dosing regimens are detailed in Tables 4A and 4B.

**TABLE 4A**

| Group | Treatment |
|---|---|
| 1 Vehicle (0.2 ml, po, qd) | + Vehicle (0.1 ml, sc, 2/wk) |
| 2 4TBPPAPC 30 mg/kg (0.2 ml, po, qd) | + Vehicle (0.1 ml, sc, 2/wk) |
| 3 2xCon4(C) 2.8 µg (0.1 ml sc, 2/wk) | + Vehicle (0.2 ml, po, qd) |
| 4 4TBPPAPC 100 mg/kg (0.2 ml, po, qd) | + Vehicle (0.1 ml sc, 2/wk) |
| 5 2xCon4(C) 14 µg (0.1 ml, sc, 2/wk) | + Vehicle (0.2 ml, po, qd) |
| 6 4TBPPAPC 30 mg/kg (0.2 ml, po, qd) 2/wk) | + 2xCon4(C) 2.8 µg (0.1 ml, sc, |
| 7 4TBPPAPC 30 mg/kg (0.2 ml, po, qd) 2/wk) | + 2xCon4(C) 14 µg (0.1 ml, sc, |
| 8 44TBPPAPC 100 mg/kg (0.2 ml, po, qd) 2/wk) | + 2xCon4(C) 2.8 µg (0.1 ml, sc, |
| 9 4TBPPAPC 100 mg/kg (0.2 ml, po, qd) 2/wk) | + 2xCon4(C) 14 µg (0.1 ml, sc, |

**TABLE 4B**

| | Optimal dose | Suboptimal dose |
|---|---|---|
| 2xCon4(C) | 14 µg/mouse, 2/wk, sc | 2.8 µg/mouse, 2/wk, sc |
| 4TBPPAPC | 100 mg/kg, qd, oral | 30 mg/kg, qd, oral |

Results are expressed and the data were analyzed as described in Example 1.

Data are presented in Figures 4A through 4D. In all of the figures data points are the group mean values and the vertical bars represent ± SEM.

The graph in Figure 4A shows the mean tumor volumes for the mice treated with the control vehicle (group 1), 4TBPPAPC (low dose) (group 2), 2xCon4(C) (low dose) (group 3), and both 4TBPPAPC (low dose) and 2xCon4(C) (low dose) (group 6).

The graph in Figure 4B shows the mean tumor volumes for the mice treated with the control vehicle (group 1), 4TBPPAPC (low dose) (group 2), 2xCon4(C) (high dose) (group 5), and both 4TBPPAPC (low dose) and 2xCon4(C) (high dose) (group 7).

The graph in Figure 4C shows the mean tumor volumes for the mice treated with the control vehicle (group 1), 2xCon4(C) (low dose) (group 3), 4TBPPAPC (high dose) (group 4), and both 4TBPPAPC (high dose) and 2xCon4(C) (low dose) (group 8).

The graph in Figure 4D shows the mean tumor volumes for the mice treated with the control vehicle (group 1), 4TBPPAPC (high dose) (group 4), 2xCon4(C) (high dose) (group 5), and both 4TBPPAPC (high dose) and 2xCon4(C) (high dose) (group 9).

All groups treated with a single agent or a combination of agents experienced a significant reduction in tumor growth compared to vehicle control (p<0.0001 for all treatment groups compared to the vehicle control group).

The combination of both agents at suboptimal doses was significantly more efficacious than suboptimal doses of either agent alone: p=0.01 for the combination compared to 4TBPPAPC, and p=0.0026 for the combination compared to 2xCon4(C).

The group treated with an optimal dose of 2xCon4(C) combined with a suboptimal dose of 4TBPPAPC experienced significantly greater reduction in tumor volume than the group treated with 4TBPPAPC alone (p<0.0001). However, this combination treatment was not significantly more efficacious than treatment with the optimal dose of 2xCon4(C) alone.

Treatment with a suboptimal dose of 2xCon4(C) combined with an optimal dose of 44TBPPAPC was significantly more efficacious than treatment with a suboptimal dose of 2xCon4(C) (p<0.0001). However, this combination was not significantly different than treatment with the optimal dose of 44TBPPAPC.

Treatment with an optimal dose of 2xCon4(C) combined with an optimal dose of 4TBPPAPC was significantly more efficacious than treatment with an optimal dose of 2xCon4(C) (p=0.0216). However, the combination treatment was not significantly more efficacious than treatment with an optimal dose of 4TBPPAPC (p=0.0527). This p value is just above the significance threshold of 0.05.

### EXAMPLE 5: 2xCon4(C) and AMG 706-Colo205 Xenografts

Xenografts of Colo205 cells injected into female athymic nude mice were treated with 2xCon4(C) alone, AMG 706 alone, or a combination of the two as described below. Tumors were induced by injecting Colo205 cells subcutaneously into female athymic nude mice (CD1 Nu/Nu), 8-10 weeks old. All of the mice were obtained from Charles River Laboratories (Raleigh, NC). Colo205 cells for injection were cultured in T-225 culture flasks. Cells were harvested from subconfluent cultures using trypsin/EDTA. The cells were greater than 95% viable by trypsin blue exclusion. The cells were suspended in 33% Matrigel™ in RPMI (free of FBS & PSG) at 10 x 10⁶ cells/ml. 2 x 10⁶ cells in 0.2 ml were injected subcutaneously into the right flank of each mouse.

On day 17 following injection the tumors averaged 200 mm³, and the mice were divided at random into four groups of 10 mice each. Group 1 received AMG 706 at a dose of 24.4 mg/kg and Fc fragment control at a dose of 2.8 µg. Group 2 received 2xCon4(C) at a dose of 2.8 µg and the pH 2 water vehicle. Group 3 received AMG 706 at a dose of 24.4 mg/kg and 2xCon4(C) at 2.8 µg. Group 4 received pH 2 water vehicle plus Fc fragment control at 2.8 µg and served as a negative control. 2xCon4(C) and the Fc fragment control groups were administered subcutaneously twice per week. AMG 706 and the pH 2 water vehicle groups were administered orally twice per day, except for days 25 and 26.

The experiment was performed "blind" in accordance with the Cancer Pharmacology Blinded Study Guidelines. Tumors were measured and body weights were determined twice each week. Tumors were measured with an electronic digital caliper. Tumor volumes were calculated as the length x width x height measurements and expressed in mm³.

The data were analyzed by repeated measures ANOVA for tumor volumes over time using STATVIEW v. 5.0.1. Scheffe's post-hoc test was used to determine p values for repeated measures from day 17 to day 37 on all four groups, and also from day 17 to day 48 for groups 1, 2, and 3.

Treatment with 2xCon4(C) in combination with AMG 706 (group 3) significantly inhibited tumor growth compared to either AMG 706 (group 1) or 2xCon4(C) (group 2) (p<0.05 on day 37). In addition, 2xCon4(C) in combination with AMG 706 and the single agent group, 2xCon4(C), significantly inhibited tumor growth compared to the control group on day 37 (p<0.05). Administration of AMG 706 and 2xCon4(C) together (group 3) significantly enhanced tumor inhibition compared to either agent by itself (p<0.0005 on day 48). The time course of tumor size for all four groups is shown in Figure 5.

No effect was observed on the body weight of any of the groups.

### COMPARATIVE EXAMPLE 6: 2xCon4(C) (2.8 µg) and Avastin™ (30 µg)

Xenografts of Colo205 cells injected into female athymic nude mice were treated with 2xCon4(C) alone, Avastin™, or a combination of the two as described below.

Tumors were induced by injecting Colo205 cells subcutaneously into female athymic nude mice (CD1 Nu/Nu), 8-10 weeks old. All of the mice were obtained from Charles River Laboratories (Raleigh, NC). Colo205 cells for injection were cultured in T-225 culture flasks. Cells were harvested from subconfluent cultures using trypsin/EDTA. The cells were greater than 95% viable by trypsin blue exclusion. The cells were resuspended in 33% Matrigel™ in RPMI (free of FBS & PSG) at 10 x 10⁶ /ml. 2 x 10⁶ cells in 0.2 ml were injected subcutaneously into the right flank of each mouse.

On day 16 after injection the tumors averaged 300 mm³, and the mice were divided at random into four groups of 10 mice each. Group 1 received IgG1 at a dose of 30 µg and Fc fragment control at a dose of 2.8 µg and served as a negative control. Group 2 received Avastin™ at a dose of 30 µg and Fc fragment control at 2.8 µg. Group 3 received 2xCon4(C) at a dose of 2.8 µg and human IgG1 at a dose of 30 µg. Group 4 received 2xCon4(C) at a dose of 2.8 µg and Avastin™ at 30 µg. All mice receiving 2xCon4(C) and the Fc fragment control were administered subcutaneously twice per week. All mice receiving Avastin™ and the human IgG1 were administered IF twice per week.

The experiment was performed "blind" in accordance with the Cancer Pharmacology Blinded Study Guidelines. Tumors were measured and body weights were determined twice each week. Tumors were measured with an electronic digital caliper. Tumor volumes were calculated as the length x width x height measurements and expressed in mm³.

The data were analyzed by repeated measures ANOVA for tumor volumes over time using STATVIEW v. 5.0.1. Scheffe's post-hoc test was used to determine p values for repeated measures from day 16 to day 51 for all groups.

Treatment with Avastin™ alone (group 2) exhibited significant tumor inhibition compared to the control (group 1) (p<0.001). Treatment with 2xCon4(c) alone resulted in a 24% inhibition of tumor growth that was not statistically significant compared to the control. Treatment with 2xCon4(C) and Avastin™ together resulted in significant tumor inhibition compared to the control (p<0.0001) and compared to treatment with 2xCon4(C) alone (p<0.0001). Treatment with 2xCon4(C) and Avastin™ together exhibited slightly greater inhibition of tumor growth than did Avastin™ alone, but the difference was not statistically significant. The time course of tumor size for all groups in shown in Figure 6.

No effect was observed on the body weight of any of the groups.

### COMPARATIVE EXAMPLE 7: 2xCon4(C) (2.8 µg) and Avastin™ (10 µg)

Xenografts of Colo205 cells injected into female athymic nude mice were treated with 2xCon4(C) alone, Avastin™, or a combination of the two as described below.

Tumors were induced by injecting Colo205 cells subcutaneously into female athymic nude mice (CD1 Nu/Nu), 8-10 weeks old. All of the mice were obtained from Charles River Laboratories (Raleigh, NC). Cells were harvested from subconfluent cultures using trypsin/EDTA. The cells were greater than 95% viable by trypsin blue exclusion. 2 x 10⁶ cells in 0.2 ml were injected subcutaneously into the right flank of each mouse.

On day 14 after injection the tumors averaged 250 mm³, and the mice were divided at random into four groups of 10 mice each. Group 1 received IgG1 at a dose of 10 µg and Fc fragment control at a dose of 2.8 µg and served as a negative control. Group 2 received Avastin™ at a dose of 10 µg and Fc fragment control at 2.8 µg. Group 3 received 2xCon4(C) at a dose of 2.8 µg and human IgG1 at a dose of 10 µg. Group 4 received 2xCon4(C) at a dose of 2.8 pug and Avastin™ at 10 µg. All mice receiving 2xCon4(C) and the Fc fragment control were administered subcutaneously twice per week. All mice receiving Avastin™ and the human IgG1 were administered IP twice per week.

The experiment was performed "blind" in accordance with the Cancer Pharmacology Blinded Study Guidelines. Tumors were measured and body weights were determined twice each week. Tumors were measured with an electronic digital caliper. Tumor volumes were calculated as the length x width x height measurements and expressed in mm³.

The data were analyzed by repeated measures ANOVA for tumor volumes over time using STATVIEW v. 5.0.1. Scheffe's post-hoc test was used to determine p values for repeated measures from day 14 to day 42 for all groups.

Treatment with Avastin™ alone exhibited a 18% reduction in tumor growth compared to the control. Treatment with 2xCon4(c) alone resulted in a 22% inhibition of tumor growth. Treatment with 2xCon4(C) and Avastin™ together resulted in significant tumor inhibition compared to the control (p<0.0001) and compared to treatment with 2xCon4(C) alone or Avastin™ alone (p<0.0001 for both). The time course of tumor size for all groups in shown in Figure 7.

No effect was observed on the body weight of any of the groups.

From the foregoing description, one skilled in the art can easily ascertain the essential characteristics of this invention, and without departing from the spirit and scope thereof, can make various changes and modifications of the invention to adapt it to various usages and conditions.

## Claims

1. A combination of
(a) 2xCon4(C), and
(b) AMG 706 and pharmaceutically acceptable salts thereof; for use in treating a solid tumour in a subject.

2. Pharmaceutically acceptable formulations of 2xCon4(C), in combination with AMG 706 for use according to claim 1.

3. 2xCon4(C) and AMG706 for use according to claim 1 or 2 wherein the solid tumour is adenocarcinoma.

4. 2xCon4(C) and AMG706 for use according to any one of claims 1 to 3, wherein the subject is human.

5. 2xCon4(C) and AMG706 for use according to any one of claims 1 to 3, wherein (a) and (b) are administered at different times, sequentially.

6. 2xCon4(C) and AMG706 for use according to any one of claims 1 to 3, wherein (a) and (b) are administered at the same time.

7. 2xCon4(C) and AMG706 for use according to claim 3, wherein the adenocarcinoma is colon cancer.

8. 2xCon4(C) and AMG706 for use according to claim 1 wherein AMG 706 is administered at a dose of 25 mg to 125 mg once a day.

## Patentansprüche

1. Kombination aus
(a) 2xCon4(C) und
(b) AMG 706 sowie pharmazeutisch verträglichen Salzen davon;
zur Verwendung bei der Behandlung eines soliden Tumors in einem Subjekt.

2. Pharmazeutisch verträgliche Formulierungen von 2xCon4(C), in Kombination mit AMG 706 zur Verwendung nach Anspruch 1.

3. 2xCon4(C) und AMG 706 zur Verwendung nach Anspruch 1 oder 2, wobei es sich bei dem soliden Tumor um ein Adenokarzinom handelt.

4. 2xCon4(C) und AMG 706 zur Verwendung nach einem der Ansprüche 1 bis 3, wobei es sich bei dem Subjekt um einen Menschen handelt.

5. 2xCon4(C) und AMG 706 zur Verwendung nach einem der Ansprüche 1 bis 3, wobei (a) und (b) zu unterschiedlichen Zeitpunkten auf sequenzielle Weise verabreicht werden.

6. 2xCon4(C) und AMG 706 zur Verwendung nach einem der Ansprüche 1 bis 3, wobei (a) und (b) zur gleichen Zeit verabreicht werden.

7. 2xCon4(C) und AMG 706 zur Verwendung nach Anspruch 3, wobei es sich bei dem Adenokarzinom um ein Kolonkarzinom handelt.

8. 2xCon4(C) und AMG 706 zur Verwendung nach Anspruch 1, wobei AMG 706 in einer Dosis von zwischen 25 mg und 125 mg ein Mal pro Tag verabreicht wird.

## Revendications

1. Combinaison de
(a) 2xCon4(C), et de
(b) AMG 706 et des sels pharmaceutiquement acceptables de ceux-ci ; pour leur utilisation dans le traitement d'une tumeur solide chez un sujet.

2. Formulations pharmaceutiquement acceptables de 2xCon4(C), en combinaison avec AMG 706, pour leur utilisation selon la revendication 1.

3. 2xCon4(C) et AMG 706, pour leur utilisation selon la revendication 1 ou 2, la tumeur solide étant un adénocarcinome.

4. 2xCon4(C) et AMG 706, pour leur utilisation selon l'une quelconque des revendications 1 à 3, le sujet étant un être humain.

5. 2xCon4(C) et AMG 706, pour leur utilisation selon l'une quelconque des revendications 1 à 3, où (a) et (b) sont administrés séquentiellement, à différents moments.

6. 2xCon4(C) et AMG 706, pour leur utilisation selon l'une quelconque des revendications 1 à 3, où (a) et (b) sont administrés en même temps.

7. 2xCon4(C) et AMG 706, pour leur utilisation selon la revendication 3, l'adénocarcinome étant un cancer du côlon.

8. 2xCon4(C) et AMG 706, pour leur utilisation selon la revendication 1, AMG 706 étant administré en une dose de 25 mg à 125 mg une fois par jour.
